# EUROPEAN PATENT SPECIFICATION

(11) **EP 1 811 980 B1**
(45) Date of publication and mention of the grant of the patent: **01.06.2011**
(21) Application number: 05808487.2
(22) Date of filing: 30.09.2005
(51) Int. Cl.: A23L 1/00

(54) **SEALED, EDIBLE FILM STRIP PACKETS AND METHODS OF MAKING AND USING THEM**
VERSIEGELTE ESSBARE FOLIENSTREIFENPAKETE UND VERFAHREN ZU IHRER HERSTELLUNG UND VERWENDUNG
PAQUETS SCELLES COMPOSES DE BANDES PELLICULAIRES COMESTIBLES ET PROCEDES DE FABRICATION ET D'UTILISATION DE CES PAQUETS

(30) Priority: 30.09.2004 US 614399 P
(43) Date of publication of application: 01.08.2007
(73) Proprietor: The Hershey Company, Hershey, Pennsylvania 17033 (US)
(72) Inventor: CARROLL, Thomas, J., Mechanicsburg, PA 17050 (US); KUMIEGA, Steven, M., Hummelstown, PA 17036 (US); PAUL, Brian, J., Millersburg, PA 17061 (US); HUZINEC, Robert, J., Hummelstown, PA 17036 (US); WARD, Philip, C., Palmyra, PA 17078 (US); KLINE, Mary, Ellen, Lancaster, PA 17601 (US)
(74) Representative: ZBM Patents
(86) International application number: PCT/US2005/035190
(87) International publication number: WO 2006/039487

(56) References cited:
- EP-A- 0 783 839
- EP-A- 1 151 672
- WO-A-00/45794
- WO-A-02/19987
- US-A- 2 901 357
- US-A- 3 554 767
- US-A1- 2003 224 090
- 'Handbook of Pharmaceutical Excipients', 1986, AMERICAN PHARMACEUTICAL ASSOCIATION AND THE PHARMACEUTICAL SOCIETY OF GREAT BRITAIN

## Description

### Reference to Related Applications

This application claims priority benefit of U.S. Provisional Application 60/614,399, filed September 30, 2004, the entire contents of which are hereby incorporated by reference.

### Field of the Invention and Introduction

The invention relates to edible films, novel packets made of the films, and novel methods to produce a sealed packet containing a variety of confectionery products and/or other appropriate and sealable compositions. In a preferred method and aspect, the invention can be used to produce a confectionery product comprising an edible film and sealable components, whereby the sealable components can be released from a packet made of the edible film upon ingestion or contact with the tongue or mouth. In other embodiments, the film can be used to package a variety of compositions for human or animal consumption or use.

In one aspect, the products and methods of the invention allow those in the art to produce stable packets for the delivery of a variety of confectionery ingredients. In particular, the packet center can include components for delivering sensations or active agents, such as cool, hot, fizzing, breath-freshening, germ-killing, or mouth-watering, which are released from the packet once put into the mouth. In one aspect, at least one high cooling component is included in the center of the packet by using an edible ingredient having a negative heat of solution, such as xylitol or other edible polyol. In general, the products may have various flavors, good stability, and an acceptable shelf life at room temperature. They can be designed and produced in a variety of shapes and sizes.

### Description of Related Art

Various edible films have been produced, sold in the market, and discussed in the art. Recently, these films have been made into breath-freshening strips that dissolve when placed in the mouth. The breath freshening strips are designed and produced as a single layer. Others have suggested the use of films as a food ingredient or packaging material for food ingredients, especially in the baking industry (*see* Watson, J., "Soluble films for small ingredient control," The Bakers Digest, June 1970, pp 42-43). The Watson document mentions rolled film or tape as a manufacturing advantage in packaging using existing packaging mechanisms. Still others have suggested the use of edible films in delivering pharmaceutically active agents (*see* U.S. Patent 5,948,430).

However, the challenge for the successful use of edible film or polymer products is stability and proper functioning during storage and intended use. Typically, this means films are manufactured in controlled environments and produced as dry or very low moisture films. The low moisture characteristics also aid in dissolving the film in the mouth.

As described in detail below, in one aspect the inventors have expanded on the edible film concept by employing processes to control the water retained in the film in order to create self-sealing films that can be used to enclose and deliver tastes, sensations, and/or other compositions. Thus, the invention provides novel uses for edible films and provides new production methods for making packeted products having advantageous properties.

### Summary of the Invention

The present invention relates to a product and its process of manufacture that, in an important but non-limiting aspect, results in a stable, edible packet or sachet. The packet comprises an edible film, or two edible films of optionally differing flavors, colors, and/or compositions, that can be formed into a packet and sealed to enclose a confectionery or other encapsulated, dehydrated, or dry ingredient or composition. US 2003224090 discloses to snacks comprising one or more orally soluble edible films that disintegrate upon placement in the mouth. The film may comprise HPMC and CMC, and also an humectant. In particular an encapsulated snack prepared by forming an envelope with the film, placing a center composition inside and then sealing the envelope via a heat sealing or using a binder for sealing.

EP-A-0783839 discloses an edible sachet containing a soluble food product in an edible film comprising carboxymethylcellulose, a plasticizer and gelatin. In order to form the sachet, the edible film may be heat-sealed around. Gelatin is needed in order to make a thermo-sealable film.

WO 02/19987 discloses a dry powder film forming composition for use in coating pharmaceutical tablets, food, and confectionery products, comprising a film forming agent including a powdered cellulosic polymer and and gum acacia, and a powdered edible plasticizer. The formulation is intended to be put into solution and applied to a tablet or other substrate without an extended waiting period.

WO 00/45794 discloses an edible, hardenable, prompt release coating composition comprising (a) microcrystalline cellulose, (b) a film forming amount of carrageenan, and (c) at least one of a strengthening polymer and a plasticizer, wherein said coating composition does not, when ingested or placed in an aqueous medium, significantly retard release of active ingredients from a substrate to which said coating is applied, said strengthening polymer being hydroxypropylmethyl cellulose. The coating composition is to be sprayed on the substrate.

US-A-3554767 discloses a sugar coated confectionery consisting essentially of an inner candy core coated, the coat containing carboxymethyl cellulose. The coat is applied on a substrate by forming a coating suspension.

EP-A-1151672 discloses a confectionery product comprising a core and a filling enclosed within the casing wherein the casing is a protective confectionery material and the filling comprises a major amount of monosaccharide polyol in a crystalline anhydrous powder form chosen among polyols having a cooling effect.

In one embodiment, the invention provides a method of making a confectionery, such as a method of making a confectionery packet capable of dissolving in the mouth, where the method involves providing an edible film and forming the edible film into a pre-packet shape. In general, any available edible polymer or food grade polymer can be used to produce the edible film and many edible films are already known in the art. The pre-packet shape refers to the ability to create a folded or sealed packet from one strip or piece of film or to the ability to create a packet by placing two strips or pieces of film next to each other or in contact with each other. Thus, for example, packets can be sealed along all four sides of two rectangular shapes placed together or along three sides of one folded rectangular shape. Commonly used packaging methods can be used or adapted for use to create the pre-packet shape and final packet once the pre-packet shape is sealed. There are many options in designing a shape to the final packet product, such as rectangles, squares, hearts, circles, stars, etc., and the final shape can actually be cut from a packet of another shape, thus creating packets with holes or other design features. The invention is not limited by the way the packets can be shaped, sized or contoured. The examples below detail a rectangular shape of about 24 mm by about 30 mm, or a square of about 24 mm by about 24 mm, that is used as a confectionary product, and a larger size packet, sachet, or pouch for use in dissolution to form a beverage or solution, but these are merely exemplary.

The method also involves filling the pre-packet shape with a center composition and sealing the pre-packet shape to enclose the center composition. The method of sealing can encompass applying water or moisture directly to the surfaces to be sealed, applying heat or a hot element with or without pressure to the surface to be sealed, a combination of these, and/or the use of water-based adhesives, food grade adhesives, and/or edible adhesives, or any other available method. In general, the films selected for use can be sealed to enclose the center composition and then remain sealed at room temperature. The preferred polymers for use in preparing or providing the edible film are hydroxypropylmethyl cellulose (HPMC), carboxymethyl cellulose (CMC), a mixture of HPMC and CMC, pectin, and combinations of any or all of these. Other edible films previously used in the art, and which may also be used in the invention, are those having one or more of cellulose ethers, starch, hydroxypropylated starch, corn zein, wheat gluten, soy protein, pullulan, sodium alginate, and milk proteins.

In a preferred embodiment of the packet or sachet the comprises a combination of HPMC and CMC in the film, at least two different apparent viscosities for HPMC may be used in a mixture of different types of the HPMC low viscosity polymer. The at least two different types of HPMC used can have an apparent viscosity of about less than 0.005 Pa·s (5 centipoise) and about 0.1 Pa·s (100 centipoise) for a 2% aqueous solution of HPMC at 20 degrees C. Other examples taken from the embodiments listed or exemplified can be selected, including those referred to in the Tables below. Where the edible packet has a film comprising two different types of HPMC, the ratio of the different types (high viscosity to low viscosity, for example) can be about 70/30 to about 30/70, or any ratio in between. Other ratios of different types of HPMC, such as about 90/10 through about 10/90, can also be selected and used. In addition, in any embodiment of the films noted here, the film for the edible packet may contain a CMC level of about 5% by weight and/or the film can be about 0.0457 mm (1.8 mil) to about 0.0559mm (2.2 mil) in thickness and/or the film can contain about 7% to about 9% retained water by weight. Furthermore, the center composition if these packets can comprises one or more of xylitol, erythritol, mannitol, sorbitol, lactitol, isomaltulose, maltitol, or powdered hydrogenated glucose syrup. The center composition can also comprise a flavoring agent and optionally a flow agent.

The center composition can comprise a number of functional or flavor components and can actually be a single ingredient if desired. Typically, the center composition is chosen as a dried, dehydrated, glass encapsulated, and/or encapsulated ingredient that will not dissolve the film on contact or absorb water to dissolve or dry the film. In a preferred embodiment, the center composition is edible and has a water activity substantially the same as the film or films being used, or is dried to an extent that drying or wetting of the film or films from contact with the center composition is substantially avoided. There are many available methods to dry, dehydrate or encapsulate components and compositions for use in this regard (*see, for example* U.S. patent document 2004/0081735 or international patent document WO 97/16078).

In a preferred embodiment, the center composition comprises a sugar alcohol or other edible compound or food ingredient that has a negative heat of solution. When such a compound contacts the tongue or inside of the mouth after the edible film dissolves, a cooling sensation results. A center composition can also be selected to increase the solubility of the edible film and one of the advantageous properties of a center composition comprising xylitol is that the film is more easily dissolved and has an improved mouth feel. While a cooling sensate is a preferred center composition, many other sensates and ingredients can be selected and used. One preferred embodiment employs a center composition comprising xylitol, which has a negative heat of solution and produces a cooling sensation when it contacts the tongue or the moist membranes of the mouth. Other polyols can create similar cooling sensations and can be used instead or in various combinations, including erythritol, mannitol, sorbitol, and maltitol. As used here, a sensate or sensate composition or ingredient is a compound or composition that can be ingested or used in the mouth and creates a physical or physiological sensation. Exemplary sensations are high cooling, cooling, hot, tingling, mouth-watering, and freshening. Many compounds and ingredients are already available for such sensations, including menthol, menthone glycerol ketal, (-)-Menthoxypropane-1,2-diol, 3-(1-Menthoxy)-2-methylpropane-1,2-diol, p-Menthane-3,8-diol, 2-Isopropyl-N,2,3-trimethylbutyramide, (-)-Menthyl lactate for cooling sensates, for example.

The center composition in any aspect of the invention can also contain the ingredients for producing a beverage once dissolved. For example, dried milk and cocoa powder can be used in the center composition to produce a chocolate and/or cocoa packet that can be added to milk or water. The center composition can also contain herbal, nutriceutical, pharmaceutical, veterinary, vitamin, anti-bacterial, or other compounds or compositions. Functionally, the center composition can comprise any compound, ingredient, or compositions that can remain dried and exist stably while enclosed within the edible film selected. Some compounds, ingredients, or compositions can be spray dried, encapsulated, plated, or adsorbed to produce it in an acceptable condition or improve its ability to be sealed within the edible film. One of skill in the art is familiar with many such methods and any available method can be used.

Various flavoring agents, flavors, or sweeteners can also be used in the center composition. For example, mints, spearmint, peppermint, wintergreen, cinnamon, menthol, anise, thymol, eucalyptol, fruits, citrus, melon, berry, vanilla, orange, raspberry, and chocolate flavors can be used. The flavoring agents to be used for these and many other flavors are known in the art. In one embodiment, a center composition comprising menthol, thymol, eucalyptol and methyl salicylate can be used, and in others the flavoring agent(s) can be mixed with a carrier or polyol to prepare a solid.

In another aspect, the invention comprises a method of making an edible packet where the packet is composed of an edible film, wherein the film has a retained moisture content of about 3% to about 15%, preferably about 4% to about 12%, or about 5% to about 10%, more preferably about 7% to about 10%, or about 6% to about 10%, water by weight (as measured by available techniques after about one hour of equilibration), and/or wherein the film is capable of being sealed against itself or another film. In another aspect, the final product produced has a retained moisture content of about 6% to about 10% as measured by the Karl Fischer method or Fourier Transform IR (FTIR) methods after about one hour equilibration. In fact, the examples and tables below indicate numerous moisture levels for particular films of the invention, and any range of moisture levels selected from two or more of those listed can be selected as a range for a particular self-sealing film of the invention, including 6.09 to 9.29% and 7.35 to 8.54% for example. In effect, and without limiting the invention to any particular means or mechanism of sealing edible films, controlling the level of retained water in the film allows one to use heat and pressure along desired sides or points of the film to seal the film to itself or to another film. A variety of polymers can be selected or tested to produce an edible film with the desired retained water level or desired characteristics of self-sealing. By self-sealing, what is meant is a film that can be sealed against itself, or against another film of similar or identical composition, in order to enclose a center composition. The film or films can be formed into a pre-packet shape with one or more strips of film. In this and in any other aspect of the invention, if two or more different films are used, the films can contain differing flavor and/or color components to create a mixture of flavors and colors in the edible films used. Similarly, a single packet can be separated into two or more sub-packets with more than one center composition in each enclosed area. Combination of flavors and colors, for example, can also be combined in the two or more films and two or more sub-packets used for a particular product.

Returning to the general method, the pre-packet shape using the sealable film or film with desired retained water level can be filled with a center composition, wherein the center composition is edible or designed for human or veterinary or oral use or consumption. The sealing of the pre-packet shape can be by applying heat and pressure and/or by applying water-based adhesive, such as a gelatin solution or sugar based adhesive (*see* for example, US 6,613,378 for edible adhesives) at the sealing sites to form a closed packet with the center composition enclosed within the sealed packet. Again, as noted above, sub-packet areas can produce products with more than one sealed center composition and the size and shape of the packets or sachets can vary as desired.

In one general embodiment, the method of making the packet or sachet employs a film having a low viscosity polymer, and a preferred embodiment is carboxymethyl cellulose (CMC), hydroxypropylmethyl cellulose (HPMC), and combinations of these. Glycerin, polypropylene glycol, 1,3-butylene diol, and/or glycerol monostearate, as well as any combination of these including propylene glycol combined with glycerin, and a hydrocolloid or gum can also be used in the preferred embodiment of the combined HPMC and CMC film. A hydrocolloid or gum can function to soften or make the film more elastic or pliable during production or aid in the ability of the low viscosity polymer to form a thin film or dryable film that can be manipulated during processing and sealed. A variety of hydrocolloid or gum products or functional equivalents can be selected for this purpose, including certain edible plasticizers such as the preferred glycerin. In general, the hydrocolloid or gum can be selected from one or more of carrageenan, pullulan, gelatin, gum arabic, locust bean gum, guar gum, xanthan, starch, maltodextrin, gum ghatti, gum karaya, gum tragacanth, dextran, konjac flour, arabinogalactan, gellan gum, agar-agar, furcellaran, and alginate. Carrageenan is preferred. In more general aspects of the film, any suitable water soluble, edible polymer can be used to form the film or films, such as, for example, CMC, methylcellulose, HPMC, guar gum, locust bean gum, xanthan gum, hydrolyzed gums, carrageenan, tamarind, agar-agar, konjac, arabinogalactan, larch arabinogalactan, betaglucan, algins, propylene glycol, polyvinyl pyrrolidone, methycrylate copolymer, carboxyvinyl copolymers, levan, elsinan, pullulan, pectins, curdlon, chitosan, gum arabic, corn starch, waxy maize starch, high amylose corn starch, potato starch, tapioca starch, rice starch, wheat starch, modified starches, acid modified starches, bleached starches, oxidized starches, esterified starches, etherified starches, crosslinked starches, and protein or protein compositions such as albumen, gelatin, casein, salts of casein, whey, wheat gluten, zein, and protein derived from soybeans. The film can also include one or more sweeteners, such as the preferred sucralose and/or Acesulfame K, but may also contain one or more other intense sweeteners. A non-limiting list of exemplary sweeteners includes sugarless sweeteners, sugar alcohols such as sorbitol, mannitol, xylitol, isomalt, lactitol, hydrogenated starch hydrolysates (HSH), maltitol, sucralose, aspartame, N-substituted APM derivatives, neotame, salts of acesulfame, alitame, saccharin and its salts, cyclamic acid and its salts, stevioside, glycyrrhizin, dihydrochalones, thaumatin, and monellin. In general, it is preferred to use an intense sweetener or a sweetener having a multiplicity of sweetness compared to sucrose so that the sweetness in the film balances the flavor of the film as it dissolves in the mouth. Again, the film can further comprise one or more flavoring agents or ingredients and one or more coloring agents or ingredients, including one or more fruit flavors and one or more of the sensate compounds, sweeteners, or compositions mentioned above for the center composition.

In other aspects, the invention includes the packets produced from any of the mentioned methods or other packets comprising an edible film sealing a center composition. As noted, the center composition in a confectionery aspect of the packet comprises xylitol. However, the center composition can also be or contain one or more of: erythritol, mannitol, sorbitol, lactitol, isomaltulose, maltitol, powdered hydrogenated glucose syrup, a flavoring agent, such as spearmint, peppermint, wintergreen, cinnamon, menthol, anise, thymol, eucalyptol, fruit, citrus, melon, and berry flavors. Combinations with sugar and/or other sweeteners may also be used, including those comprising one or more of a saccharide-containing component, dextrose, sucrose, fructose, lactose, maltose, dextrin, invert sugar, levulose, galactose, and corn syrup solids and the like. The combinations can also include starches, gelatins, and thickening agents. The center composition can also comprises a breath freshening agent, anti-bacterial agent, pharmaceutical agent, or nutriceutical agent. Also, the center composition can comprise a savory or fat-based composition, such as a composition having one or more of dark chocolate, milk chocolate, bittersweet chocolate, semisweet chocolate, or white chocolate or cocoa powder, or dried or dehydrated components for producing a tea, a tea-based beverage, a soup, a chili, and/or salty, spicy, or other savory ingredients. Thus, the invention specifically includes a packet or sachet formed to enclose a center composition to make a beverage or other edible solution when placed or dissolved in water, milk, or other liquid.

In another aspect of the invention, a method for producing an edible film is provided, where the edible film is selected to have a certain retained water level and/or a self-sealing characteristic. The method involves selecting an edible film, varying a plasticizer and/or hydrocolloid or gum component in the film to produce a film that can be self-sealing or have a desired retained water level. A preferred retained water level is from about 5% to about 10% water by weight. Other ingredients in the film can also be varied to produce the desired film, as well as the heating or cooking conditions, drying conditions, and drying method. One of skill in the art is familiar with many types of edible films to use for this purpose as well as the related methods for producing these films. Without limiting the invention to any particular explanation of how and why it works and provides advantages, the inventors believe that certain aspects of the films and methods described contain or employ enough residual water to allow the film to seal on itself. Using a CMC film, or a HPMC film, or the combined CMC and HPMC film or embodiment, a specifically preferred range of about 4% to about 12%, or about 6% to about 10%, or 7% to about 10%, or about 8% to about 10% (or others ranges discussed above or taken from values in the examples or tables below) by weight residual water is desirable. To date, no suitable, edible or food grade adhesive exists for sealing the edible films as described here. Providing a self-sealing film, thus, addresses a need in the art and provides manufacturing advantages by eliminating the need for an adhesive to form a packet. Furthermore, the packets of the invention are preferably of a type that are stable at room temperature and can be produced as individual units that do not stick to each other, melt, or break open during storage or handling. Existing edible films are typically very dry, brittle, and adhere to one another.

Throughout this disclosure, applicants refer to journal articles, patent documents, published references, web pages, and other sources of information. One skilled in the art can use the entire contents of any of the cited sources of information to make and use aspects of this invention. Each and every cited source of information is specifically incorporated herein by reference in its entirety. Portions of these sources may be included in this document as allowed or required. However, the meaning of any term or phrase specifically defined or explained in this disclosure shall not be modified by the content of any of the sources. The description and examples that follow are merely exemplary of the scope of this invention and content of this disclosure and do not limit the scope of the invention. In fact, one skilled in the art can devise and construct numerous modifications to the examples listed below without departing from the scope of this invention.

### Brief Description of the Drawings

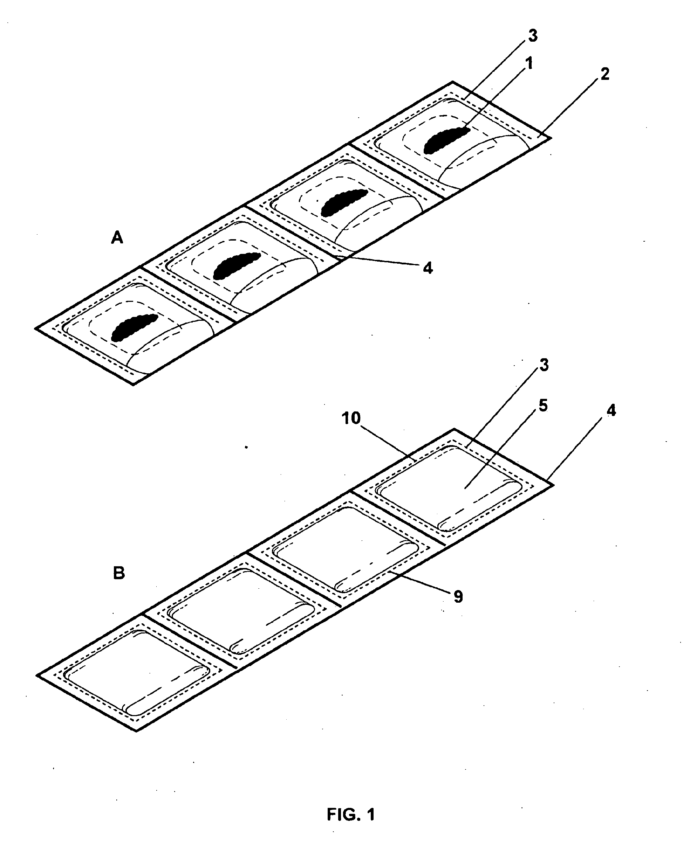

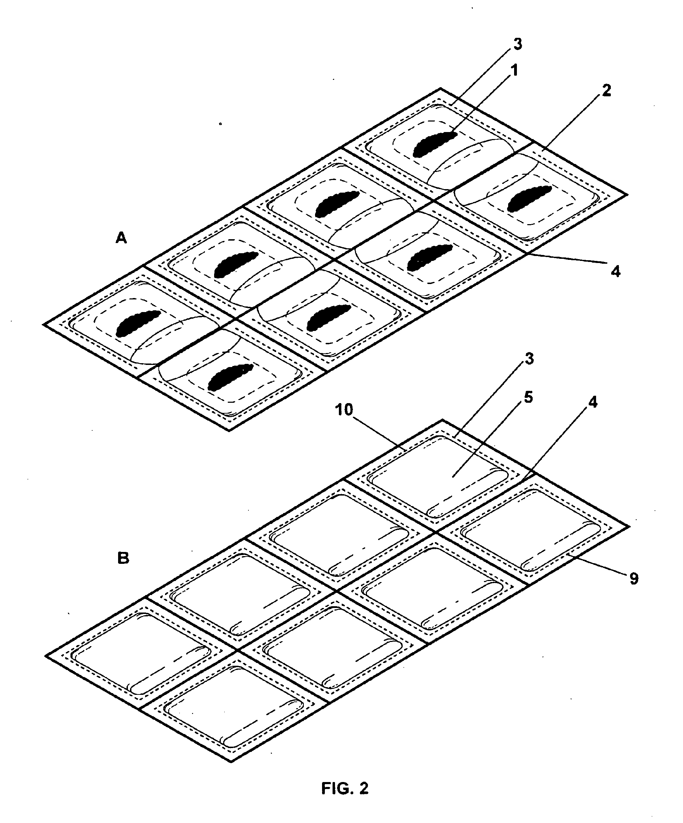

Figures 1A, 1B, 2A, 2B, and 3 show preferred examples of the packet or sachet of the invention. In Figures 1A and 2A the center composition (1) is filled or placed in or on a film (2) having a pre-packet area defined by the dotted line (3). Line (4) represents the cutting point or separation point between two packets. In Figures 1A and 2A, two layers or strips of film have been placed together to form the packet and are ready for sealing around center composition (1). Figures 1B and 2B show a single film layer or strip, where point (5) represents the area where the center composition can be placed. Figures 1A and 1B depict a strip of film where a row of packets is formed. Figures 2A and 2B depict a strip of film where two rows of packets are formed. Multiple rows can be employed. Dotted lines (9) and (10) may either or both represent areas where the single strip of film can be folded over onto itself to create a pre-packet form, thus reducing the points or sides that must be sealed. Line (4) again represents the dividing or cutting line between two packets. A preferred packet or sachet is produced as a final product of about 24 mm by about 24 mm square, with approximately 3 mm for the sealing area on the external sides of each square.

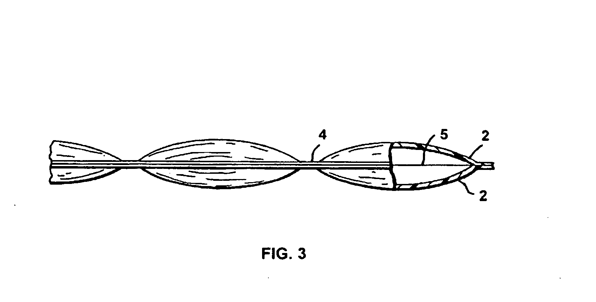

Figure 3 shows a side view of a packet, where center composition (not visible), is within two strips of film at area (5). Each side of the packet is represented by a film (2) or a film (7) as the two layers or strips of film forming the packet. As noted, the shape, sizing, and contours of the packet can be altered from those shown in the Drawings.

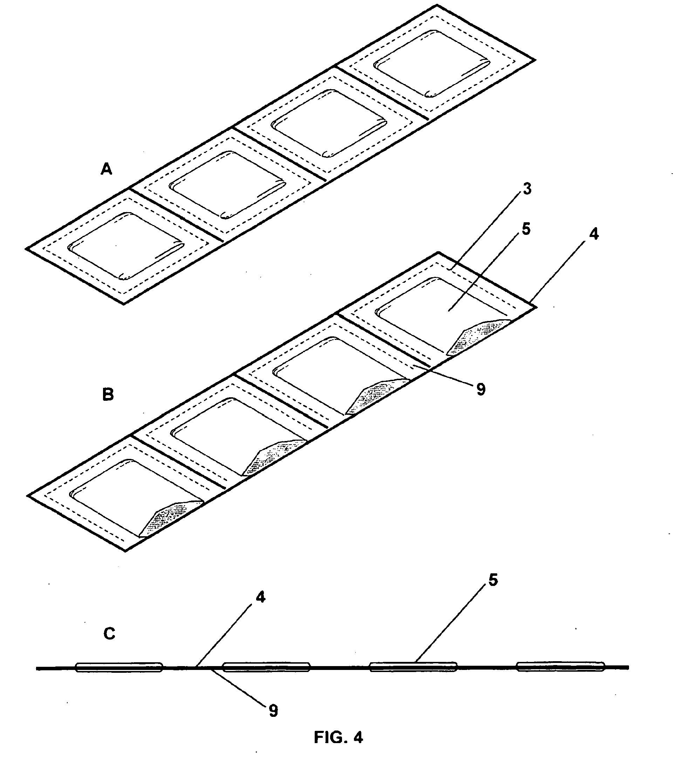

Figure 4A-C shows differing views of exemplary prototype packets or sachets. The center composition (5) can be seen in Figure 4B, and sealing area (3) is about 3 mm for a 24 mm by 24 mm packet. Heating and pressure sealing along lines in area (9) separate the center compositions and allow the four-packet form to be cut into four separate packets. Figure 4C depicts an approximated side view of the packet.

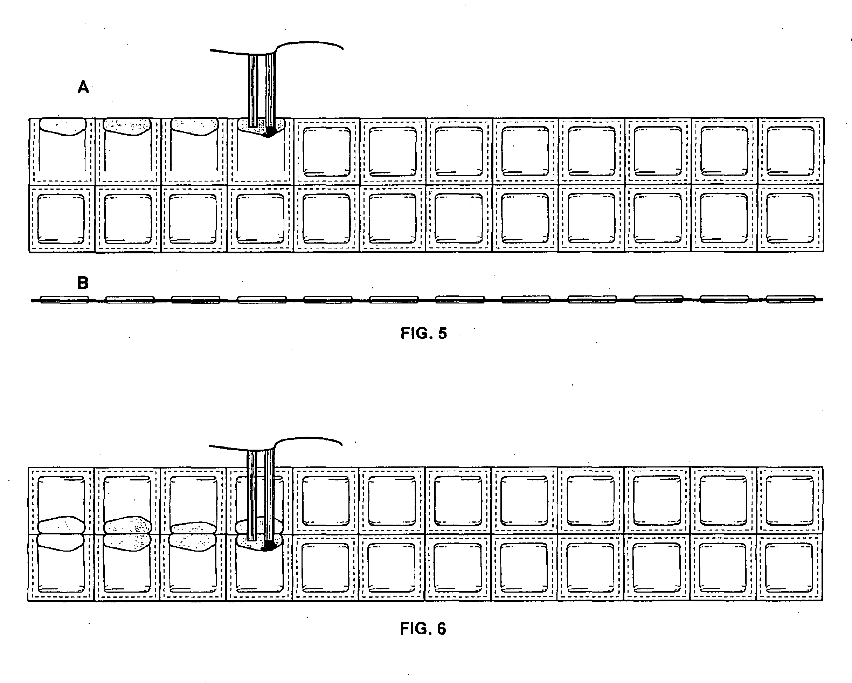

Figures 5A and B and 6 depict the orientation of a twelve-packet form that can be used in production methods. Once three sides of each packet or sachet are formed, the center composition can be filled and heat and pressure applied, where filling and vacuum tubes can optionally be inserted into each pre-packet shape as in Figures 5A and 6. In Figure 5A, eight sets of sealed and formed packets are shown while four sets are in the process of being filled and sealed. As the final side of the packet is formed to seal the center composition, the three sides of the pre-packet form are sealed for the next row of packets (not shown). Figure 5B shows a side view of the sealed packets. In Figure 6, the forth side is sealed and the twelve-packet form cut into 12 individual packets. A sheet with multiple twelve-packet rows can be used in production methods, however any desired number of rows can be used according to the invention.

### Detailed Description of Exemplary Embodiments

As noted above, in one aspect of the invention a product comprising a packet is provided. The packet can be filled or can enclose a center composition and among the many types of center compositions one of skill in the art can select from are those that contain one or more polyols, such as xylitol, erythritol, mannitol, sorbitol, lactitol, isomaltulose, maltitol, or similarly a powdered hydrogenated glucose syrup. Other ingredients in the center composition can comprise: one or more sugars, such as sucrose, dextrose, maltose, and fructose; one or more acidulants, such as citric, malic, tartaric, fumaric, acetic, or ascorbic acid (either straight or encapsulated); one or more salts, such as sodium, potassium, sodium bicarbonate; one or more nutraceuticals, such as vitamin complexes, herbals, anti-bacterials, germ killing agents; one or more flavors, such as mints including spearmint, peppermint, and wintergreen, cinnamon, menthol, anise, thymol, eucalyptol; one or more cooling agents, such as sensates for hot, cold, tingling, mouth-watering, freshening sensations; one or more fruits, such as citrus, melon, berry, pitted, and seeded fruits; one or more sweeteners or intense sweeteners, such as aspartame, Acesulfame potassium (Ace-K), neotame, saccharin, sucralose, glycirhizin and its salts such as monoammonium glycirhizinate, cyclamates, monellin, and talin; one or more savory flavors, such as meat, poultry, vegetable; one or more chocolate or indulgent flavors; one or more spicy flavors; one or more vanilla flavors; one or more additives, such as fruit powder, fat-based creams, fat mimetic-based creams, fruit pieces, or dried fruit; and/or one or more functional or breath freshening compounds, such as zinc compounds or extracts to enhance the breath-freshening efficacy, polyphenols, antioxidants, mushroom extract, tooth-whitening agents, and over-the-counter medicaments or other pharmaceutical products. The flavors can be in the form of a liquid, an encapsulated product or ingredient, a film- based speck or glitterant, a spray dried product or ingredient, a capsule, glass matrix, agglomerated, or extruded product or ingredient, or a plated or absorbed/adsorbed product or ingredient. Similarly, the center composition can include one or more of: nonfat dry milk powder; whey; corn syrup solids; vitamins and minerals; salt; natural and artificial flavors (optionally spray dried); cream powders; vanillin; sucrose; partially hydrogenated soybean oil; artificial colorings (optionally in granular or powder form); cocoa powder; alkalized cocoa powder; cornstarch; soya lecithin; preservatives such as sulfur dioxide, flow agents such as calcium, calcium stearate, silicon dioxide, calcium mono silicate; or sodium benzoate; dehydrated ingredients, such as marshmallows, vegetables, or fruit; baking chips; and/or edible glitter.

The edible or food grade films that can be used in the methods or products of the invention are many. One skilled in the art is already familiar with methods to produce these films, such as those described in U.S. patent documents 6,419,903; 6,177,096; 6,284,264; 5,747,648; 5,948,430; 4,876,092; 5,047,244; 5,948,430; 6,231,957; 6,419,903; 6,740,332; 2001/0022964; 2001/0046511; 2001/0051186; 2004/0115137; 2004/0087467; 2004/0086546; 2004/0096569; 2004/0136922; and international publications WO 00/18365; WO 02/43657. Each of these documents is specifically incorporated herein by reference and one of skill in the art can use and rely on the contents of these documents to make and use films as described herein, and to select ingredients for the films and center compositions as described herein. However, nothing in these documents will change a term defined or explained in this disclosure. For example, the films having different combinations of flavors, colors, additives, and/or ingredients can be selected from those mentioned in the foregoing documents. Also, the films used can have printed designs or other information or decorations placed upon them or incorporated into them.

In one embodiment of a non-acid containing film with extremely quick dissolving properties, the products are prepared from a very low viscosity water soluble polymer (0.01-0.02 Pa·s (10-20 cps) in 1% solution) in combination with a water soluble gel forming sulfated hydrocolloid. CMC is a preferred low viscosity polymer and carrageenan is a preferred sulfated hydrocolloid. Iota carrageenan is the most preferred product. In one example, CMC is used at a level of 30% to 40% in the finished film and carrageenan is used at a level of 3% to 5% in the finished film assuming a final film product moisture of 5%. CMC contributes to film forming and quick dissolution in the mouth, while carrageenan provides structure and strength for production and during handling. In addition to the above, an emulsifier (preferably glycerol monostearate) and a polyalcohol (preferably glycerol or glycerin) are used at a ratio of 1:1 and can range at a level from about 5% to 15% of the finished film. Flavor components comprised of flavorful compounds and carriers can vary up to about 60% of the final weight of the film. Intense artificial sweeteners are added as desired to adjust flavor. Colors are also added at the end as desired. Table 1 below relates to certain CMC films of the invention.

| **TABLE 1** | | | | | | | | | | |
|---|---|---|---|---|---|---|---|---|---|---|
| **EX** | **Ing.1** | **Ing.2** | **Ing.3** | **Ing.4** | **Ing.5** | **Ing.6** | **Ing.7** | **Ing.8** | **Proc** | **Dissol** |
| Pepmint | | | | | | | | | | |
| #1 | 35.52 | 4.44 | 11.84 | 11.84 | 0 | 35.52 | 0.6 | 0.24 | 1 | 4 |
| #2 | 36.8 | 4.6 | 12.26 | 12.26 | 1.59 | 31.64 | 0.61 | 0.24 | 1 | 4 |
| #3 | 30.3 | 3.78 | 10.1 | 10.1 | 1.64 | 43.27 | 0.63 | 0.21 | 2 | 4 |
| #4 | 42.02 | 6.36 | 5.53 | 5.53 | 1.77 | 38.03 | 0.55 | 0.22 | 3 | 3 |
| #5 | 38.7 | 4.14 | 11.06 | 5.53 | 1.77 | 38.03 | 0.55 | 0.22 | 2 | 3 |
| #6 | 40.28 | 4.85 | 15.53 | 0 | 1.84 | 36.8 | 0.58 | 0.12 | 3 | 3 |
| Raspber | | | | | | | | | | |
| #7 | 34.64 | 4.33 | 11.55 | 11.55 | 1.38 | 35.74 | 0.58 | 0.23 | 2 | 4 |

where the Example (EX) # for various peppermint (Pepmint) and raspberry (Raspber) flavored films are shown, and where Ing.1 is CMC; Ing.2 is carrageenan; Ing.3 is glycerin; Ing.4 is glycerin monosulfate; Ing.5 is artificial sweetener; Ing.6 is flavor; Ing.7 is emulsifier; Ing.8 is color; "Proc" refers to the relative processing ability, for example to manipulate and use the film in producing a packet, and is related to flexibility and stability on a scale of 1-4, with 4 being the highest or best processing characteristics and 1 being the lowest; "Dissol" refers to the dissolvability of the film when placed in the mouth and relates to a pleasing mouth feel for the film, with 4 being the highest or best and 1 being the lowest. In certain examples, a film of about 0.0381 mm (1.5 mil), or between about 0.0381mm (1.5 mil) and about 0.0762mm (3.0 mil) in thickness is selected.

In the case of acid or high acid containing films, the low viscosity hydrocolloid is a relatively low molecular weight high methoxylated pectin (59% to 65% methoxylated) and the sulfated hydrocolloid is agar-agar. Polymer and hydrocolloid amounts and ranges are the same as above but preferred levels are 45% and 5%, respectively. The emulsifier:polyalcohol level is preferred at 6.4% and the flavor component is set at 14%. In addition, desirable acids such as citric, acetic, malic, fumaric, tartaric, adipic, etc., and a buffer salt designed to maximize sourness is added such that the acid:buffer ratio is about 1:1 and the acid level can vary from 10% to 20%. Preferred buffers are sodium lactate, sodium polyphate, sodium tartrate and potassium tartrate.

To achieve an optional spotted color effect in films, 0.1% to 10% of hydroxypropyl cellulose (HPC) is added to the product based on final product weight. HPC precipitates out of solution during the heating step when the batch temperature rises above 38°C (100° F). This causes a color pooling effect in the finished films which appears as colored dots once the film is dried.

In another embodiment, a film of HPMC and CMC and carrageenan is produced to provide improved sealing qualities and elasticity properties for mechanized production on a variety of machines available in the art. Preferred machines utilize vertical film processing and filling steps. In Table 2 below, a number of films that can be produced with a combination of HPMC and CMC according to the invention.

**TABLE 2**

| **EX** | **H1** | **H2** | **H3** | **CMC** | **MC** | **Sw** | **Gly** | **PGI** | **Car** | **Flav** | **Emul** | **Col** | **P** | **D** | **%M** |
|---|---|---|---|---|---|---|---|---|---|---|---|---|---|---|---|
| Pepmt | | | | | | | | | | | | | | | |
| #1 | 29.16 | 9.72 | | 4.94 | 2.03 | 2.02 | 18.03 | | 5.26 | 28.09 | 0.64 | 0.12 | 4 | 4 | 7.84 |
| #2 | 19.14 | 19.44 | | 4.94 | 2.03 | 2.02 | 18.03 | | 5.26 | 28.09 | 0.64 | 0.12 | 2 | 2 | 7.65 |
| #3 | 19.77 | | 19.77 | 4.38 | 1.95 | 1.98 | 16.34 | | 5.21 | 27.89 | 0.62 | 0.12 | 1 | 4 | 8.89 |
| #4 | 15.82 | | 23.72 | 4.38 | 1.95 | 1.98 | 16.34 | | 5.21 | 27.89 | 0.62 | 0.12 | 3 | 3 | 8.54 |
| #5 | 11.86 | | 27.68 | 4.38 | 1.95 | 1.98 | 16.34 | | 5.21 | 27.89 | 0.62 | 0.12 | 4 | 2 | 7.35 |
| #6 | 23.72 | | 15.82 | 4.38 | 1.95 | 1.98 | 16.34 | | 5.21 | 27.89 | 0.62 | 0.12 | 2 | 4 | 8.1 |
| #7 | 27.68 | | 11.86 | 4.38 | 1.95 | 1.98 | 16.34 | | 5.21 | 27.89 | 0.62 | 0.12 | 1 | 4 | 8.75 |
| #8 | 23.94 | 15.96 | | 5.07 | 2.08 | 2.02 | 15.97 | | 5.4 | 28.81 | 0.66 | 0.09 | 3 | 3 | |
| #9 | 25.93 | 13.96 | | 5.07 | 2.08 | 2.02 | 15.97 | | 5.4 | 28.81 | 0.66 | 0.09 | 3 | 3 | |
| #10 | 27.93 | 11.97 | | 5.07 | 2.08 | 2.02 | 15.97 | | 5.4 | 28.81 | 0.66 | 0.09 | 2 | 4 | |
| | | | | | | | | | | | | | | | |
| Wntgr | | | | | | | | | | | | | | | |
| #11 | 23.01 | | 23.01 | 2.51 | 2.01 | 2.12 | 16.54 | 2.01 | 5.93 | 22.12 | 0.72 | 0.02 | 4 | 3 | 9.29 |
| #12 | 18.41 | | 27.61 | 2.51 | 2.01 | 2.12 | 16.54 | 2.01 | 5.93 | 22.12 | 0.72 | 0.02 | 3 | 3 | 6.09 |
| #13 | 13.81 | | 32.21 | 2.51 | 2.01 | 2.12 | 16.54 | 2.01 | 5.93 | 22.12 | 0.72 | 0.02 | 3 | 2 | 6.72 |
| #14 | 27.61 | | 18.41 | 2.51 | 2.01 | 2.12 | 16.54 | 2.01 | 5.93 | 22.12 | 0.72 | 0.02 | 3 | 3 | 7.88 |
| #15 | 32.21 | | 13.81 | 2.51 | 2.01 | 2.12 | 16.54 | 2.01 | 5.93 | 22.12 | 0.72 | 0.02 | 2 | 4 | 8.43 |
| #16 | 24.79 | 16.53 | | 4.72 | 2.15 | 2 | 17.32 | | 5.59 | 26.19 | 0.68 | 0.02 | 3 | 3 | |
| #17 | 26.85 | 14.46 | | 4.72 | 2.15 | 2 | 17.32 | | 5.59 | 26.19 | 0.68 | 0.02 | 3 | 3 | |
| #18 | 28.91 | 12.39 | | 4.72 | 2.15 | 2 | 17.32 | | 5.59 | 26.19 | 0.68 | 0.02 | 2 | 4 | |

where the Example (EX) # for various peppermint (Pepmt) and wintergreen (Wntgr) flavored films are shown, and where the ingredients listed are present in weight %. The ingredients are: H1 and H2 and H3 refer to three different types or molecular weights of HPMC that can be used; CMC is carboxymethyl cellulose; MC is microcrystalline cellulose; Sw is artificial sweetener(s); Gly is glycerin; PGl is propylene glycol; Car is carrageenan; Flav is flavoring agent(s); Emul is emulsifier(s); Col is coloring agent(s). The properties of the films and their ability to produce desirable or optimum edible packets or sachets can also be tested. In Table 2, P is processing ability, as noted above; D is dissolvability as noted above; and %M is moisture content expressed as weight percent and measured after one hour equilibration at 50% R.H. and 21°C (70° F). In general, the thickness is about 0.0457mm (1.8 mil), or about 0.0457 mm (1.8 mil) to about 0.0559mm (2.2 mil), or about 0.0381mm (1.5 mil) to about 0.762mm (3.0 mil). The combined HPMC and CMC films produce advantageous results in terms of the processability and dissolvability properties for making packets or sachets.

The information in this document can be used to produce a variety of films to make edible packets or sachets as described using any of the low viscosity edible polymers to make the films, and any combination of them. However, when a particular content of CMC is selected, for example about 5% by weight CMC or as discussed in the Examples, a combination with HPMC can be preferred. In fact, the use of HPMC solutions having differing apparent viscosity measurements as indicated from the supplier can be tested for the most desirable mouth feel, dissolving characteristics in the mouth, and processing ability in a production or manufacturing environment. HPMC, while a low viscosity edible polymer, can be prepared and is available in a variety of relative viscosity levels. In Table 2, the H1 column refers to a low viscosity HPMC product, such as one with an apparent viscosity of less than 0.005 Pa·s (5 centipoise) in a 2% solution at 20 degrees C. The H2 column refers to a relatively high viscosity HPMC, with an apparent viscosity of about 0.1 Pa·s (100 centipoise) in a 2% solution at 20 degrees C. The H3 column refers to a relative medium level of viscosity for HPMC of about 0.045 Pa·s (45 centipoise) in a 2% solution at 20 degrees C. Mixtures of HPMC that can be used with CMC include HPMC mixtures with ratios of about 10/90 high viscosity HPMC to low viscosity HPMC, or about 20/80, or about 30/70, or about 40/60, or about 50/50, or about 60/40, or about 70/30, or about 80/20, or about 90/10, with preferred ratios between 30/70 and 70/30. Generally, the medium viscosity HPMC can replace the high viscosity HPMC in the ratios noted here.

One skilled in the art is familiar with rolled film techniques or other machines for using edible films in order to place two films in contact with one another to enclose and seal the center compositions described here. The machines used in packaging can be adapted for this purpose. Thus, the films described here can be produced as sheets or rolls and then used in a manufacturing machine to fill the center compositions and seal the center composition within the film. In one specific embodiment, 10 grams or more of a chocolate, savory or other food ingredient or flavored composition can be used as the center composition for a relatively large packet. In another specific embodiment, two rectangular film strips or pieces of about 24 mm by about 24 mm, or about 24 mm by about 18 mm are used. Where a HPMC/CMC or carboxylmethyl cellulose film is used as described below, each piece of film weighs about 06 grams but can be 04 to about .10 grams in the final packet or sachet where the film is about 0.0457mm (1.8 mil) to about 0.0559mm (2.2 mil) in thickness. Together, these two pieces of film can be used to enclose about .25 to about .50 grams of center composition comprising xylitol, or about .10 grams to about .50 grams of center composition. A center composition comprising xylitol and a flow agent is preferred. As noted in the Summary, this is merely an example of the many shapes and sizes that can be selected for the packets of the invention. The center composition in this example has about 90% to about 95%, or about 95% to about 99.5% (by dry weight) xylitol or erythritol, about 0.1% to about 8.0% flavor or glass encapsulated flavor, and about .10% to .50% intense sweetener, such as sucralose or neotame.

Examples of film where mechanical properties and production scale-up may not be of particular import include those prepared by adding water, glycerol, emulsifier, buffers (if desired), and an intense sweetener into a batching vessel. The mix is heated to about 82°C (180°F). Under high shear using a Silverson mixer or other homogenizer, the water-soluble polymers are added slowly into the vortex such that good hydration occurs and no "fish eyes" are formed. The fully hydrated mix is transferred to a heating vessel and heated with constant stirring until 85% solids or a good film-forming consistency is achieved. The mixture is cooled to below 46°C (115° F) at which time acid (if desired), and flavors are mixed-in with constant stirring. After this, a film is cast on a flat surface using a Gardner knife for even distribution. Films can be cast such that final thickness is between 50 and 100 microns. Thinner films will dissolve quickest when consumed. The cast films are allowed to dry at room temperature overnight before cutting, filling, and sealing.

Typical production methods include a casting process, where water-soluble polymers and plasticizers or other ingredients are mixed and then metered into appropriate amounts and dried in a tunnel oven. Another method involves extruding, where ingredients are mixed and forced into a tube, heated, and extruded out an orifice under pressure. Another process involves calendaring, where the extruding process above is use except that the heated mixture is fed into a series of heated rolls which press the extruded slabs into a film.

In general, edible films must be safe to eat and can preferably be made of products that are generally recognized as safe (GRAS) by the U.S. FDA or other government regulatory body. Also, edible films are typically made of at least one polymer or film-forming material. As noted herein, there are many different polymers that can be used. In general, a low viscosity polymer(s) or combinations of polymers is preferred for the invention. However, high viscosity polymers, as known in the art, can be used and/or diluted to produce satisfactory films.

In the processing of the polymer composition into a film, the viscosity chosen can be important as can the heating to a percent solid content. In a preferred example shown below where mechanical properties and production scale-up may not be of particular import, the film ingredients are mixed and heated until about 85% solids is reached. This % of solids in the CMC example results in a final film that possesses the desired stability, ability to be handled, and water retention level. In other embodiments, where a different polymer is used or a combination having a different viscosity, the heating and mixing step may be modified in order to achieve the proper solids content that leads to a stable, adequately flexible, and self-sealing film. General categories include carbohydrates, proteins, solid lipids or waxes, or resins. Examples of carbohydrate polymers include CMC, HPMC, CMC combined with HPMC, hydroxypropyl cellulose (HPC), methylcellulose, pectin, dextrins, pullulan, and alginates. Proteins and protein compositions that can be used include corn zein, soy protein isolates, albumin, collagen, and whey. Waxes and resins that can be used include beeswax, carnuba wax, and shellac. Combining different components can enhance certain characteristics of the final film product.

From a functional perspective, the one or more polymers or components of the film used should take into consideration both the viscosity characteristics of the polymer or composition and the ability to pour and/or produce a stable film, and/or a film that is not too brittle to be handled and packaged. Typically, low molecular weight polymers produce relatively weak films and of low viscosity. These films can be strengthened by adding additional polymers and/or one or more hydrocolloids, or made more flexible by adding one or more plasticizers. A plasticizer is a substance which, when added to another material, increases the flexibility or plasticity of that material. One of skill in the art is familiar with many plasticizers or hydrocolloids that can be used in accordance with the invention, and some include sorbitol, glycerol or glycerine, propylene glycol, 1,3-butylene diol, and polyethylene glycol and the like.

In general, a low viscosity polymer or combination of polymers is preferred for the invention. However, high viscosity polymers can be used and/or diluted to produce satisfactory films. In the processing of the polymer composition into a film noted above, the film ingredients are mixed and heated until about 85% solids is reached. This % of solids in the carboxymethyl cellulose example results in a final film that possesses 5%-10% retained water by weight. As noted above, one of the preferred polymers, CMC, is a quick dissolving polymer with a viscosity of 0.01-0.02 Pa·s (10-20 cps) at 1% solution. In other embodiments, where a different polymer is used or a combination having a different viscosity, the heating and mixing step may need to be modified in order to achieve the proper solids content that leads to a stable, adequately flexible, and self-sealing film. HPMC and CMC combinations may offer additional or improved flexibility and mechanical properties for production.

Similarly, high acid films can be selected and used and similar factors affecting the viscosity and heating conditions can be considered. Again, hydrocolloids can be added to high acid films to produce the desired final characteristics.

Since the films are designed to produce a packet and enclose a center composition, the type of center composition desired can be taken into account when selecting the film composition and its characteristics. Certain polymers or polymer compositions may be more desirable for center compositions that do not absorb water, while others may be more desirable for center compositions that are prone to water absorption. The film can optionally be coated or sprayed on one or both sides to affect the stability, flexibility, or water retention level, and/or the moisture migration characteristics. One of skill in the art is familiar with compounds and compositions to reduce the water migration that can be selected and used.

### Examples

**Example 1:** Edible Films with Desired Retained Water Levels

An edible film is produced using the following ingredients: 30.0 grams CMC (carboxymethyl cellulose) TIC15; 3.75 grams Agar 379; 10.0 grams Glycerol; 10.0 grams GMS (glycerol monostearate); 0.9 to 1.3 grams sucralose; and 750.0 grams water. These ingredients are hydrated by mixing on a Silverson-type mixer. The mixed ingredients are cooked at 177-204°C (350-400°F) until greater than or about 85% solids is achieved - approximately 20-30 minutes. The resulting mix is removed from the stove unit and optionally 2.8 grams of Tween (emulsifier) is added. Desired flavoring and/or coloring can also be added. In one embodiment, 10 ml. to 15 ml. of peppermint oil is used after cooling. This mixture is spread to a desired thinness as a slab on a lab bench table (for 24 hr. process), or placed on glass sheets and stove at 62-65°C (144-149°F) for approximately one hour. The edges can be trimmed prior to removing the finished/dried film. By adding or modifying the content of a hydrocolloid or gum such as carrageenan, and/or modifying the content of glycerol and/or glycerol monostearate, and/or modifying the drying conditions or thinness of the film, a desired retained water level can be monitored by detecting the weight of the film and comparing it to the theoretical for a certain desired water content. Similarly, employing a different polymer, such as pectin, or hydroylpropyl methylcellulose, or other polymer, the desired retained water level can be produced.

For an exemplary cooling sensate center, a sealable composition of one or more sugar alcohols can be used, such as the preferred xylitol. Xylitol powder (Xylitol CM 170) is sifted prior to being blended with other ingredients. Exemplary batches can be selected from those with one or more polyols (here xylitol used) alone, xylitol plus coloring, xylitol plus coloring and flavoring, and/or xylitol plus flavoring. For example: 100 grams of sifted xylitol; 96.0 grams of xylitol plus 4.0 grams of 1 mm gelatin encapsulated peppermint flavor capsules; 98.0 grams of xylitol plus 2.0 grams of 1 mm gelatin encapsulated peppermint flavor capsules; 90.82 grams of Xylitol CM170, 0.3 grams of sweetener such as Aspartame; 8.53 grams of glass encapsulated mint flavor, 0.2 grams of citric acid powder, and 0.15 grams of vanillin. Optionally, a flow agent or anti-caking agent can be added, such as Hubersorb 600 at about 0.5 grams, or silicon dioxide as appropriate levels.

The packets can be cut to any desired shape, size, or adapted for any manufacturing process. In one example, the film is cut to 24mm x 36 mm rectangles and filled with about .5g of the center composition. Two layers of film are used to sandwich the entire center composition and the edges of the film are sealed by direct application of a moist swab or preferably by applying heat and pressure for a desired period of time as is commonly performed with a sealing device or roller. One of skill in the art will appreciate that too much heating time or too high of a temperature will burn the film. In general, the methods of the invention use the residual water in a film to seal the packet and the center composition within the two pieces of film. After drying, the films together form a packet around the center composition.

### Example 2: Fruit Films

For the following films, hot tap water is mixed with glycerol, and polymer is mixed with agar. Using a Silverson mixer slowly add GMS then polymer/agar mixture, then add sweeteners. Pour mix into pot to cook on agitated lab cooker at about 260°C (500F). Put into agitator in chuck. Stir solution and add color, for example 5 drops for watermelon flavor and color. Cook 15 to 30 min. until temperature reaches 96°C (204F). Cooking time is approximately 23 minutes. Take off heat and cool under agitation to 38°C (<100F). Add optional flavors, colors, and acids. Make film by pouring on flat surface or benchtop and monitor with calibrated unit. Let dry overnight. Use razor blade to cut straight ends and loosen one end and pull film off surface.

### Raspberry Film

| | |
|---|---|
| Ingredient | Amount, g |
| Water | 89760.0 |
| CMC-15 | 3170.0 |
| GP 379 | 390.0 |
| Glycerin | 1200.0 |
| GMS | 1200.0 |
| Acesulfame K | 60.0 |
| Sucralose | 60.0 |
| Raspberry Flavor | 1170.0 |
| Raspberry Flavor | 1530.0 |
| Peach Flavor | 590.0 |
| Cooling compound #1 | 420.0 |
| Cooling compound #2 | 420.0 |
| Color (Red) | 30.0 |

### Watermelon Film

| Ingredient | Amount, g |
|---|---|
| Water | 85180.0 |
| Pectin | |
| (Unipectin, PG, 769S) | 7950.0 |
| Agar (TIC, RS-100) | 340.0 |
| Glycerin | 1140.0 |
| GMS | 1140.0 |
| Acesulfame K | 60.0 |
| Sucralose (Micronized) | 60.0 |
| Watermelon Flavor | 1420.0 |
| Buff. Lactic Acid (BF S/30) | 1360.0 |
| Malic Acid (Granular) | 1360.0 |
| Color (Red) | 30.0 |

The following two films are produced to a desired retained water level by measuring the dry weight of the film.

### Cherry Film

| Ingredient | Amount, g |
|---|---|
| Water | 930.0 |
| Pectin DD-Slow Set | 70.0 |
| GMS | 5.0 |
| Glycerol | 10.0 |
| Flavor | 25.0 |
| Acesulfame K | 15.0 |

### Cherry Film (Trial #1A)

| Ingredient | Amount, g |
|---|---|
| Water | 930.0 |
| Pectin 769s | 70.0 |
| Rhodigel EZ | 6.5 |
| GMS | 5.0 |
| Glycerol | 10.0 |
| Flavor | 25.0 |
| Acesulfame K | 5.0 |

The xylitol-containing packets using CMC film produces products that easily dissolve in the mouth and present a noticeable cooling sensation. The texture and feel of the film is acceptable. The stability of the packets at ambient or room temperature with no special packaging is greater than 30 days, and the packets do not adhere to one another or lose their contents.

### Example 3: Beverage Pouch

The packets of the invention can be produced and sized to accommodate a mix or composition for producing a beverage when placed in water, milk, or other liquid. As noted above, the amount of center composition used can vary depending on the desired final beverage or solution. For a hot chocolate example, about 10 or more grams of cocoa powder-containing center composition can be used. Depending on the desired final beverage or solution, the film used to prepare the pouch or packet can be made without any flavoring.

In a preferred example of a hot chocolate pouch that can placed into hot water or hot milk to produce a hot chocolate beverage, the following ingredients and percent composition can be used in formulating a center composition to be sealed within a film, preferably a film having no flavor agents or flavoring:

| | |
|---|---|
| Artificial sweeteners | 0.45% |
| Flavor | 2.13% |
| Cocoa powder | 16.70% |
| Non-dairy creamer | 77.49% |
| Vanillin | 0.34% |
| Salt | 1.22% |
| Gum | 1.67% |
| | |

In a specific example of a hot cocoa beverage pouch, the following ingredients, percent composition, and weight in grams can be used, again preferably with a film having no flavor agents or flavorings:

| **Hot cocoa pouch** | | **grams** |
|---|---|---|
| Ingredients | % | 13.000 |
| Acesulfame K | 0.180% | 0.023 |
| Aspartame | 0.270% | 0.035 |
| Chocolate flavor | 0.800% | 0.104 |
| Dark Red Cocoa Pwdr | 2.500% | 0.325 |
| Dutched Cocoa Pwdr. | 14.200% | 1.846 |
| Non-dairy creamer | 77.490% | 10.074 |
| Cream powder flavor | 0.830% | 0.108 |
| Vanillin | 0.340% | 0.044 |
| salt, flake | 1.220% | 0.159 |
| Guar Gum | 1.670% | 0.217 |
| Choc, Enhancer | 0.500% | 0.065 |
| **Total** | 100.000% | 13.000 |

### Example 4: Edible films using HPMC, CMC and Carrageenan

An edible film is produced using the following ingredients: 78.7 grams of a solution of 15% by weight of low viscosity HPMC (DOW Chemicals), 26.3 grams of a solution of 15% by weight high viscosity HPMC (DOW Chemicals), 24.1 grams of a solution of 8.3% by weight of CMC 15 (TIC Gums), 0.82 grams of Avicel PH 105 (FMC Biopolymers), 7.30 grams of glycerin, 0.41 grams of Ace-K, and 0.41 grams of sucralose. These ingredients are combined and agitated using a Silverson-type mixer, and the ingredients are mixed to produce a homogeneous, viscous solution. In a separate container 2.13 grams of MV-406 Carrageenan (FMC Biopolymer) is mixed with 11.37 grams of peppermint flavor and 0.26 grams of Polysorbate 80, and to the resulting solution 50 grams of water is added and mixed in a Silverson-type mixer to produce a flavor emulsion. Combine the above mentioned homogeneous, viscous solution with the flavor emulsion, add a desired amount of color, and mix thoroughly. The resulting mixture is cast to a desired thickness on a glass plate coated with Mylar and placed in an oven set to 80 °C until dry. The moisture content or retained water in the dried film, after equilibrating for one hour at 50% RH at 21°C (70 degrees F), can be measured as known in the art, such as FTIR methods. Similarly, the dissolvability of the film in the mouth and the processability of the film in typical production techniques, for example handling and rolling the film, can be rated for a number of different film compositions. The Tables above list some examples of the moisture content and characteristics of films that may be desired. Generally, a relatively quickly dissolving film without residual in the mouth that decrease the pleasant mouth feel combined with a film flexibility sufficient to allow handling and bending of the film, are desired for the preferred self-sealing films of the invention.

A prototype production product was produced using the combined HPMC and CMC film noted above. An approximately 100 mm x 100 mm square of film is placed onto a metal form with sixteen packet shapes in a 4x4 pattern. After one layer of film is placed on the form, a gentle vacuum is applied underneath and a center composition comprising xylitol, a flow agent, and a flavoring agent, is carefully spooned into the center of each packet shape. Another layer of film is placed on top, and then a top sealing plate (at about 204°C (400 degrees F)) is pressed by hand over the entire form for about 5 sec to seal each of the twelve packets into a square. The packets, when placed in the mouth, immediately begin to dissolve and a pleasant cooling and mouth-watering sensation results. The film generally leaves no residues on the surfaces of the mouth. The packets produced are stable at room temperature and at typical food processing and shipping temperatures for at least six to twelve months.

The examples presented above and the contents of the application define and describe examples of the many packets, films, center compositions, pouches, and final sealed products that can be produced according to the invention. None of the examples and no part of the description should be taken as a limitation on the scope of the invention as a whole or of meaning of the following claims.

## Claims

1. An edible packet comprising an outer, self-sealable film of 0.0381 to 0.0762mm (1.5 to 3.0 thousands of an inch (mil)) in thickness, and a center composition, wherein the center composition comprises one or more ingredients that have a negative heat of solution, and the center composition is dried to be stable within the outer self-sealable film and wherein the film is dried to contain 4% to 12% retained water by weight, and wherein the film comprises hydroxypropylmethyl cellulose (HPMC) and carboxymethyl cellulose (CMC) and a humectant, and wherein the film is capable of being sealed against itself under heat and pressure and the packet is stable at room temperature and capable of dissolving when placed in the mouth to release the center composition.

2. The edible packet of claim 1, wherein the retained water is of 6% to 10%.

3. The edible packet of claim 2, wherein the film further comprises carrageenan.

4. The edible packet of claim 1-2-3 wherein the center composition comprises xylitol.

5. The edible packet of claim 4, wherein the center composition further comprises a flavoring agent and optionally a flow agent.

6. The edible packet of claim 5, wherein the center composition comprises a flavoring agent selected from one or more of: spearmint, peppermint, wintergreen, cinnamon, menthol, anise, thymol, eucalyptol, fruit, citrus, melon, or berry flavors.

7. The edible packet of claim 6, wherein the center composition comprises a flow agent.

8. The edible packet of claim 2-3 wherein the film further comprises glycerin and one or more sweeteners.

9. The edible packet of claim 2-7, wherein the film further comprises a flavoring agent.

10. The edible packet of claim 3, wherein the film further comprises an edible plasticizer, and a flavoring agent.

11. The edible packet of claim 2, wherein the film further comprises carrageenan, microcrystalline cellulose, and glycerin.

12. The edible packet of claim 2, wherein the center composition comprises one or more of: xylitol, erythritol, mannitol, sorbitol, lactitol, isomaltulose, maltitol, or powdered hydrogenated glucose syrup.

13. The edible packet of claim 12, wherein the center composition further comprises a flavoring agent and optionally a flow agent.

14. The edible packet of claim 2-13, wherein the center composition comprises a combination of ingredients that contains no sugar or bulk sweetener.

15. The edible packet of claim 2-13, wherein the center composition comprises one or more sugars or bulk sweeteners.

16. The edible packet of claim 2-13, wherein the film is 0.0457 to 0.0559 mm (1.8 mil to 2.2 mil) in thickness.

17. The edible packet of claim 16, wherein the film contains 7% to 9% retained water by weight.

18. The edible packet of claim 17, which is formed into a square or rectangular shape.

19. A method of making a confectionery packet capable of dissolving in the mouth, comprising providing an edible self-sealing film, forming the film into a pre-packet shape, filling the pre-packet shape with a center composition, and sealing the pre-packet shape to enclose the center composition by applying heat and pressure, wherein the film comprises HPMC and CMC and a humectant and is formed to 0.0381 to 0.0762mm (1.5 to 3.0 mil) in thickness, and wherein the film contains 6% to 10% retained moisture, and wherein the center composition is dried to be stable within the outer self-sealable film.

20. The method of claim 19, wherein the center composition comprises xylitol and optionally a flow agent.

21. The method of claim 19, wherein the center composition comprises one or more of: xylitol, erythritol, mannitol, sorbitol, lactitol, isomaltulose, and maltitol.

22. The method of claim 19, wherein the film comprises a glycerin and optionally a flavoring agent.

23. The method of claim 19, wherein the center composition comprises a flavoring agent.

24. The method of claim 19, wherein the film further comprises carrageenan, microcrystalline cellulose, and glycerin.

25. The method of claim 24, wherein the center composition comprises one or more of: xylitol, erythritol, mannitol, sorbitol, lactitol, isomaltulose, maltitol, or powdered hydrogenated glucose syrup.

26. The method of claim 25, wherein the center composition further comprises a flavoring agent and optionally a flow agent.

27. The method of claim 19, wherein the center composition comprises a combination of ingredients that contains no sugar or bulk sweetener.

28. The method of claim 19, wherein the center composition comprises one or more sugars or bulk sweeteners.

29. The method of claim 24, wherein the film is 0.0457 to 0.0559mm (1.8 mil to 2.2 mil) in thickness.

30. The method of claim 19, wherein the film contains 7% to 9% retained water by weight.

31. The method of claim 19, where in the packet is formed into a square or rectangular shape.

32. A method of making an edible packet, comprising: providing an edible film, wherein the film has a retained moisture content of 4% to 12%, wherein the film comprises HPMC, CMC and a humectant and is formed to 0.0381 to 0.0762 mm (1.5 to 3.0 mil) in thickness and wherein the film is capable of being self-sealed against itself or another film; forming a pre-packet shape with one or more strips of film; filling the pre-packet shape with a center composition, wherein the center composition is edible or designed for human or veterinary oral use or consumption and the center composition is dried to be stable within the outer self-sealable film, and sealing the pre-packet shape by applying heat and pressure and/or applying water-based edible adhesive at sealing sites to form a closed packet with the center composition enclosed within the sealed packet.

33. The method of claim 31, wherein the film further comprises pectin.

34. The method of claim 33, wherein the film further comprises one or more of glycerin, propylene glycol, glycerol monostearate, or an edible plasticizer.

35. The method of claim 34, wherein the film further comprises a hydrocolloid or gum.

36. The method of claim 32, wherein the film further comprises carrageenan, microcrystalline cellulose, and glycerin.

37. The method of claim 36, wherein the film further comprises one or more flavoring ingredients and one or more sweeteners.

38. The method of claim 37, wherein the film further comprises one or more coloring ingredients.

39. The method of claim 32, where the center composition comprises xylitol.

40. The method of claim 36, wherein the center composition comprises xylitol and optionally a flow agent.

41. The method of claim 32, wherein the center composition comprises one or more of: xylitol, erythritol, mannitol, sorbitol, lactitol, isomaltulose, maltitol, or powdered hydrogenated glucose syrup.

42. The method of claim 41, wherein the center composition further comprises a flavoring agent and optionally a flow agent.

43. The method of claim 32, wherein the center composition comprises a combination of ingredients with no sugar or bulk sweetener.

44. The method of claim 32; wherein the center composition comprises one or more sugars or bulk sweeteners.

45. The method of claim 32, wherein the center composition comprises a flavoring agent selected from one or more of: spearmint, peppermint, wintergreen, cinnamon, menthol, anise, thymol, eucalyptol, a fruit, citrus, melon, and berry flavors.

46. The method of claim 32; wherein the center composition comprises one or more of: menthol, thymol, eucalyptol, and methyl salicylate.

47. The method of claim 32, wherein the center composition comprises a breath freshening agent, anti-bacterial agent, pharmaceutical agent, or nutriceutical agent.

48. The method of claim 32, wherein the center composition comprises one or more of dark chocolate, milk chocolate, bittersweet chocolate, semisweet chocolate, white chocolate or cocoa powder.

49. The method of claim 48, wherein the packet is formed to enclose a center composition to make a beverage when placed in water, milk, carbonated water, or other liquid.

50. The method of claim 49, wherein the beverage is chocolate flavored.

51. The method of claim 32, wherein the packet is formed into a shape designed to be placed into the mouth.

52. The edible packet of claim 1, wherein the film comprises HPMC, CMC, and a hydrocolloid or gum.

53. The edible packet of claim 1, wherein the center composition comprises xylitol.

54. The edible packet of claim 53, wherein the center composition comprises a flavoring agent selected from one or more of: spearmint, peppermint, wintergreen, cinnamon, menthol, anise, thymol, eucalyptol, fruit, citrus, melon, or berry flavors.

55. The edible packet of claim 53, wherein the film further comprises a flavoring agent and optionally a flow agent.

56. The edible packet of claim 52, wherein the packet comprises two strips of edible film comprising HPMC and CMC sealed together by heat and pressure at a desired period of time and/or water and an amount of water-based adhesive.

57. The edible packet of claim 56, wherein the edible film further comprises carrageenan, glycerin, and one or more sweeteners.

58. The edible packet of claim 57, wherein the edible film further comprises a flavoring agent.

59. The edible packet of claim 58, wherein the center composition comprises one or more of: xylitol, erythritol, mannitol, sorbitol, lactitol, isomaltulose, and maltitol.

60. The edible packet of claim 58, wherein the center composition comprises one or more of: menthol, thymol, eucalyptol, and methyl salicylate.

61. The edible packet of claim 58, wherein the center composition comprises one or more of dark chocolate, milk chocolate, bittersweet chocolate, semisweet chocolate, white chocolate or cocoa powder.

62. The edible packet of claim 58, wherein the center composition comprises one or more of: erythritol, mannitol, sorbitol, lactitol, isomaltulose, maltitol, powdered hydrogenated glucose syrup, spearmint, peppermint, wintergreen, cinnamon, menthol, anise, thymol, eucalyptol, fruit or berry flavors, dextrose, sucrose, fructose, maltose, dextrin, invert sugar, levulose, galactose, corn syrup solids, a breath freshening agent, an anti-bacterial agent, a pharmaceutical agent, a veterinary agent, and a nutriceutical agent.

63. The edible packet of claim 1, wherein the edible film comprises one or more of CMC, HPMC, guar gum, locust bean gum, xanthan gum, hydrolyzed gums, carrageenan, tamarind, agar agar, konjac, arabinogalactan, larch arabinogalactan, betaglucan, algin, propylene glycol, polyvinyl pyrrolidone, methycrylate copolymer, carboxyvinyl copolymer, levan, elsinan, pullulan, pectin, curdlon, chitosan, gum arabic, corn starch, waxy maize starch, high amylose corn starch, potato starch, tapioca starch, rice starch, wheat starch, modified starches, acid modified starches, bleached starches, oxidized starches, esterified starches, etherified starches, crosslinked starches, and protein or protein compositions such as albumen, gelatin, casein, salts of casein, whey, wheat gluten, zein, and soybean isolates.

64. The edible packet of claim 63, wherein the film contains one or more flavoring agents.

65. The edible packet of claim 1, wherein the film is substantially free of any flavoring agent.

66. The edible packet of claim 1, wherein the film has a retained water level of about 5% to about 10%.

67. The edible packet of claim 1, wherein the center composition comprises one or more savory flavors.

68. The edible packet of claim 1, wherein the center composition comprises one or more fat-based ingredients or compositions.

69. The edible packet of claim 1, wherein the center composition comprises one of more mouth-watering agents.

70. The edible packet of claim 1, wherein the center composition comprises one or more breath-freshening agents.

71. The edible packet of claim 3 or 11, wherein at least two different types of HPMC are used with at least two different apparent viscosities.

72. The edible packet of claim 71, wherein the CMC level in the film is about 5% by weight.

73. The edible packet of claim 72, wherein two different types of HPMC are a first and second HPMC, and the first HPMC has an apparent viscosity of about less than 0.005 Pa·s (5 centipoise), and the second HPMC has an apparent viscosity of about 0.1 Pa·s (100 centipoise) or about 0.045 Pa·s (45 centipoise), where the apparent viscosity represents a 2% aqueous solution of HPMC at 20 degrees C.

74. The edible packet of claim 73, wherein the first and second HPMC are present in the ratio of 70/30 to 30/70.

75. The edible packet of claim 73, wherein the first and second HPMC are present in the ratio of 60/40 to 40/60.

76. The edible packet of claim 74, wherein the second HPMC has an apparent viscosity of about 0.1 Pa·s (100 centipoise).

77. The edible packet of claim 75; wherein the second HPMC has an apparent viscosity of about 0.1 Pa·s (100 centipoise).

78. The edible packet of claim 73, wherein the center composition comprises one or more of: xylitol, erythritol, mannitol, sorbitol, lactitol, isomaltulose, maltitol, or powdered hydrogenated glucose syrup.

79. The edible packet of claim 73, wherein the center composition further comprises a flavoring agent and optionally a flow agent.

80. The edible packet of claim 73, wherein the film is 0.0457 to 0.0559mm (1.8 mil to 2.2 mil) in thickness.

81. The edible packet of claim 73, wherein the film contains 7% to 9% retained water by weight.

## Patentansprüche

1. Essbares Paket mit einem äußeren, selbst-siegelfähigen, 0,0381 bis 0,0762 mm (1,5 bis 3,0 tausendstel Zoll (mil)) dicken Film, und einer Mischung in seinem Innern, worin die Mischung im Innern einen oder mehrere Inhaltsstoffe enthält, die eine negative Lösungswärme besitzen und die besagte Mischung getrocknet wird, damit sie in dem äußeren, selbst-siegelfähigen Film stabil ist, und worin der Film getrocknet wird, bis er 4 Gew.-% bis 12 Gew.-% Haftwasser enthält, und worin der Film HydroxypropylmethylCellulose (HPMC) und Carboxymethyl-Cellulose (CMC) und ein Feuchthaltemittel enthält, und worin der Film unter hohen Temperaturen und Druck mit sich selbst verklebt werden kann, und das Paket bei Raumtemperatur stabil und ist und sich auflösen kann, wenn es in den Mund gelegt wird, um die Mischung im Innern freizugeben.

2. Essbares Paket nach Anspruch 1, worin das Haftwasser 6% bis 10% beträgt.

3. Essbares Paket nach Anspruch 2, worin der Film weiterhin Carrageen enthält.

4. Essbares Paket nach Anspruch 2 bis 3, worin die Mischung im Innern Xylitol enthält.

5. Essbares Paket nach Anspruch 4, worin die Mischung im Innern weiterhin einen Geschmacksstoff und wahlweise ein Fließmittel enthält.

6. Essbares Paket nach Anspruch 5, worin die Mischung im Innern einen Geschmacksstoff enthält, ausgewählt aus einem oder mehreren von: Grüne-Minze-, Pfefferminz-, Wintergrün-, Zimt-, Menthol-, Anis-, Thymol-, Eucalyptol-, Frucht-, Citrus-, Melone- oder Beeren-Geschmack enthält.

7. Essbares Paket nach Anspruch 6, worin die Mischung im Innern ein Fließmittel enthält.

8. Essbares Paket nach Anspruch 2 bis 3, worin der Film weiterhin Glycerin und ein oder mehrere Süßungsmittel enthält.

9. Essbares Paket nach Anspruch 2 bis 7, worin der Film weiterhin einen Geschmacksstoff enthält.

10. Essbares Paket nach Anspruch 3, worin der Film weiterhin einen essbaren Weichmacher und einen Geschmacksstoff enthält.

11. Essbares Paket nach Anspruch 2, worin der Film weiterhin Carrageen, mikrokristalline Cellulose und Glycerin enthält.

12. Essbares Paket nach Anspruch 2, worin die Mischung im Innern einen oder mehrere dieser Stoffe enthält: Xylitol, Erythrit, Mannit, Sorbit, Lactitol, Isomaltulose, Maltit und pulverförmiger hydrierter Glukosesirup.

13. Essbares Paket nach Anspruch 12, worin die Mischung im Innern weiterhin einen Geschmacksstoff und wahlweise ein Fließmittel enthält.

14. Essbares Paket nach Anspruch 2 bis 13, worin die Mischung im Innern eine Kombination von Inhaltsstoffen umfasst, die keinen Zucker oder Zuckeraustauschstoff enthält.

15. Essbares Paket nach Anspruch 2 bis 13, worin die Mischung im Innern einen oder mehrere Zucker oder Zuckeraustauschstoffe enthält.

16. Essbares Paket nach Anspruch 2 bis 13, worin der Film 0,0457 bis 0,0559 mm (1,8 mil bis 2,2 mil) dick ist.

17. Essbares Paket nach Anspruch 16, worin der Film 7 Gew.-% bis 9 Gew.-% Haftwasser enthält.

18. Essbares Paket nach Anspruch 17, dem eine quadratische oder rechteckige Form gegeben wird.

19. Verfahren zur Herstellung eines Konfektpakets, das sich im Mund auflösen kann, einschließlich Bereitstellen eines essbaren selbst-siegelfähigen Filmes, Formen des Filmes zu einer Paket-Vorform, Füllen der Paket-Vorform mit einer Mischung, und Versiegeln der Paket-Vorform, so dass die Mischung im Innern durch Anwendung von Hitze und Druck eingeschlossen wird, worin der Film HPMC und CMC und ein Feuchthaltemittel enthält und 0,0381 bis 0,0762 mm (1,5 bis 3,0 mil) dick gestaltet wird, und worin der Film 6% bis 10% zurückgehaltene Feuchtigkeit enthält, und worin die besagte Mischung getrocknet wird, um innerhalb des äußeren, selbst-siegelfähigen Filmes stabil zu sein.

20. Verfahren nach Anspruch 19, worin die Mischung im Innern Xylitol und wahlweise ein Fließmittel enthält.

21. Verfahren nach Anspruch 19, worin die Mischung im Innern einen oder mehrere dieser Stoffe enthält: Xylitol, Erythrit, Mannit, Sorbit, Lactitol, Isomaltulose und Maltit.

22. Verfahren nach Anspruch 19, worin der Film ein Glycerin und wahlweise einen Geschmacksstoff enthält.

23. Verfahren nach Anspruch 19, worin die Mischung im Innern einen Geschmacksstoff enthält.

24. Verfahren nach Anspruch 19, worin der Film weiterhin Carrageen, mikrokristalline Cellulose und Glycerin enthält.

25. Verfahren nach Anspruch 24, worin die Mischung im Innern einen oder mehrere dieser Stoffe enthält: Xylitol, Erythrit, Mannit, Sorbit, Lactitol, Isomaltulose, Maltit und pulverförmiger hydrierter Glukosesirup.

26. Verfahren nach Anspruch 25, worin die Mischung im Innern weiterhin einen Geschmacksstoff und wahlweise ein Fließmittel enthält.

27. Verfahren nach Anspruch 19, worin die Mischung im Innern eine Kombination von Inhaltsstoffen umfasst, die keinen Zucker oder Zuckeraustauschstoff enthält.

28. Verfahren nach Anspruch 19, worin die Mischung im Innern einen oder mehrere Zucker oder Zuckeraustauschstoffe enthält.

29. Verfahren nach Anspruch 24, worin der Film 0,0457 bis 0,0559 mm (1,8 mil bis 2,2 mil) dick ist.

30. Verfahren nach Anspruch 19, worin der Film 7 Gew.-% bis 9 Gew.-% Haftwasser enthält.

31. Verfahren nach Anspruch 19, worin dem Paket eine quadratische oder rechteckige Form gegeben wird.

32. Verfahren zur Herstellung eines essbaren Pakets, mit den Verfahrensschritten: Bereitstellen eines essbaren Films, wobei der Film einen Gehalt an zurückgehaltener Feuchtigkeit von 4% bis 12% besitzt, wobei der Film HPMC, CMC und ein Feuchthaltemittel enthält und 0,0381 bis 0,0762 mm (1,5 bis 3,0 mil) dick gestaltet wird, und wobei der Film mit sich selbst oder gegen einen anderen Film versiegelt werden kann; Formen einer Paket-Vorform mit einem oder mehreren Filmstreifen; Füllen der Paket-Vorform mit einer Mischung im Innern, wobei die Mischung im Innern essbar ist oder für menschliche oder tiermedizinische orale Verwendung oder Verzehr entworfen wurde und die Mischung im Innern getrocknet wird, um in dem äußeren, selbst-siegelfähigen Film stabil zu sein; und Versiegeln der Paket-Vorform durch Anwenden von Hitze und Druck und/oder Anwendung essbaren Klebstoffes auf Wasserbasis an den zu versiegelnden Stellen, um ein geschlossenes Paket zu bilden, wobei die Mischung im Innern im versiegelten Paket eingeschlossen wird.

33. Verfahren nach Anspruch 31, worin der Film weiterhin Pektin enthält.

34. Verfahren nach Anspruch 33, worin der Film weiterhin einen oder mehrere dieser Stoffe enthält: Glycerin, Propylenglykol, Glycerinmonostearat oder einen essbaren Weichmacher.

35. Verfahren nach Anspruch 34, worin der Film weiterhin ein Hydrokolloid oder Gummi enthält.

36. Verfahren nach Anspruch 32, worin der Film weiterhin Carrageen, mikrokristalline Cellulose und Glycerin enthält.

37. Verfahren nach Anspruch 36, worin der Film weiterhin einen oder mehrere Geschmacksstoffe und ein oder mehrere Süßungsmittel enthält.

38. Verfahren nach Anspruch 37, worin der Film weiterhin einen oder mehrere Farbstoffe enthält.

39. Verfahren nach Anspruch 32, worin die Mischung im Innern Xylitol enthält.

40. Verfahren nach Anspruch 36, worin die Mischung im Innern Xylitol und wahlweise ein Fließmittel enthält.

41. Verfahren nach Anspruch 32, worin die Mischung im Innern einen oder mehrere dieser Stoffe enthält: Xylitol, Erythrit, Mannit, Sorbit, Lactitol, Isomaltulose, Maltit und pulverförmiger hydrierter Glukosesirup.

42. Verfahren nach Anspruch 41, worin die Mischung im Innern weiterhin einen Geschmacksstoff und wahlweise ein Fließmittel enthält.

43. Verfahren nach Anspruch 32, worin die Mischung im Innern eine Kombination von Inhaltsstoffen ohne Zucker oder Zuckeraustauschstoff enthält.

44. Verfahren nach Anspruch 32, worin die Mischung im Innern einen oder mehrere Zucker oder Zuckeraustauschstoffe enthält.

45. Verfahren nach Anspruch 32, worin die Mischung im Innern einen Geschmacksstoff enthält, ausgewählt aus einem oder mehreren von: Grüne-Minze-, Pfefferminz-, Wintergrün-, Zimt-, Menthol-, Anis-, Thymol-, Eucalyptol-, einem Frucht-, Citrus-, Melonen- und Beerengeschmack.

46. Verfahren nach Anspruch 32, worin die Mischung im Innern einen oder mehrere dieser Stoffe enthält: Menthol, Thymol, Eucalyptol und Methylsalicylat.

47. Verfahren nach Anspruch 32, worin die Mischung im Innern ein Atemerfrischungsmittel, einen antibakteriellen Wirkstoff, pharmazeutischen Wirkstoff, oder nutrizeutischen Wirkstoff enthält.

48. Verfahren nach Anspruch 32, worin die Mischung im Innern einen oder mehrere Stoffe der Gruppe: Bitterschokolade, Milchschokolade, Zartbitterschokolade, halbsüße Schokolade, weiße Schokolade und Kakaopulver enthält.

49. Verfahren nach Anspruch 48, worin das Paket so geformt wird, dass es eine Mischung im Innern einschließt, so dass ein Getränk entsteht, wenn es in Wasser, Milch, kohlensäurehaltiges Wasser oder eine andere Flüssigkeit gelegt wird.

50. Verfahren nach Anspruch 49, worin das Getränk nach Schokolade schmeckt.

51. Verfahren nach Anspruch 32, worin das Paket in eine Form gebracht wird, die dazu entworfen wurde, in den Mund gelegt zu werden.

52. Essbares Paket nach Anspruch 1, worin der Film weiterhin HPMC, CMC und ein Hydrokolloid oder Gummi enthält.

53. Essbares Paket nach Anspruch 1, worin die Mischung im Innern Xylitol enthält.

54. Essbares Paket nach Anspruch 53, worin die Mischung im Innern einen Geschmacksstoff enthält, ausgewählt aus einem oder mehreren von: Grüne-Minze-, Pfefferminz-, Wintergrün-, Zimt-, Menthol-, Anis-, Thymol-, Eucalyptol-, Frucht-, Citrus-, Melonen- oder Beerengeschmack.

55. Essbares Paket nach Anspruch 53, worin der Film weiterhin einen Geschmacksstoff und wahlweise ein Fließmittel enthält.

56. Essbares Paket nach Anspruch 52, wobei das Paket aus zwei Streifen essbarem HPMC und CMC enthaltenden Film besteht, die durch Hitze und Druck während einer gewünschten Zeitspanne und/oder mit Wasser und einer gewissen Menge Klebstoff auf Wasserbasis zusammengeklebt wurden.

57. Essbares Paket nach Anspruch 56, worin der essbare Film weiterhin Carrageen, Glycerin und ein oder mehrere Süßungsmittel enthält.

58. Essbares Paket nach Anspruch 57, worin der essbare Film weiterhin einen Geschmacksstoff enthält.

59. Essbares Paket nach Anspruch 58, worin die Mischung im Innern einen oder mehrere dieser Stoffe enthält: Xylitol, Erythrit, Mannit, Sorbit, Lactitol, Isomaltulose und Maltit.

60. Essbares Paket nach Anspruch 58, worin die Mischung im Innern einen oder mehrere dieser Stoffe enthält: Menthol, Thymol, Eucalyptol und Methylsalicylat.

61. Essbares Paket nach Anspruch 58, worin die Mischung im Innern einen oder mehrere Stoffe der Gruppe: Bitterschokolade, Milchschokolade, Zartbitterschokolade, halbsüße Schokolade, weiße Schokolade und Kakaopulver enthält.

62. Essbares Paket nach Anspruch 58, worin die Mischung im Innern einen oder mehrere dieser Stoffe enthält: Erythrit, Mannit, Sorbit, Lactitol, Isomaltulose, Maltit, und pulverförmiger hydrierter Glukosesirup, Grüne-Minze-, Pfefferminz-, Wintergrün-, Zimt-, Menthol-, Anis-, Thymol-, Eucalyptol-, Frucht- oder Beerengeschmack, Dextrose, Saccharose, Fructose, Maltose, Dextrine, Invertzucker, Lävulose, Galactose, Maissirupfeststoffe, ein Atemerfrischungsmittel, ein antibakterieller Wirkstoff, ein pharmazeutischer Wirkstoff, ein tiermedizinischer Wirkstoff und ein nutrizeutischer Wirkstoff.

63. Essbares Paket nach Anspruch 1, worin der essbare Film einen oder mehrere der folgenden Stoffe enthält: CMC, HPMC, Guargummi, Johannisbrotkernmehl, Xanthan, hydrolysierte Gummis, Carrageen, Tamarinde, Agar-Agar, Konjac, Arabinogalactan, Lärchenarabinogalactan, Beta-Glucan, Algin, Propylenglykol, Polyvinylpyrrolidon, Methacrylat-Copolymer, Carboxyvinyl-Copolymer, Lävan, Elsinan, Pullulan, Pektin, Kurdlan, Chitosan, Gummi arabicum, Maisstärke, Wachsmaisstärke, amylosereiche Maisstärke, Kartoffelstärke, Tapiokastärke, Reisstärke, Weizenstärke, modifizierte Stärken, säuremodifizierte Stärken, gebleichte Stärken, oxidierte Stärken, veresterte Stärken, veretherte Stärken, vernetzte Stärken, und Protein oder Proteinverbindungen wie zum Beispiel Albumin, Gelatine, Casein, Caseinsalze, Molke, Weizengluten, Zein und Sojabohnen-Isolate.

64. Essbares Paket nach Anspruch 63, worin der Film einen oder mehrere Geschmacksstoffe enthält.

65. Essbares Paket nach Anspruch 1, worin der Film im Wesentlichen frei von jeglichen Geschmacksstoffen ist.

66. Essbares Paket nach Anspruch 1, worin der Film ein Haftwassergehalt von zirka 5% bis zirka 10% besitzt.

67. Essbares Paket nach Anspruch 1, worin die Mischung im Innern einen oder mehrere Geschmacksstoffe enthält.

68. Essbares Paket nach Anspruch 1, worin die Mischung im Innern eine oder mehrere Zutaten oder Mischungen auf Fettbasis enthält.

69. Essbares Paket nach Anspruch 1, worin die Mischung im Innern ein oder mehrere die Speichelproduktion anregende Mittel enthält.

70. Essbares Paket nach Anspruch 1, worin die Mischung im Innern ein oder mehrere Atemerfrischungsmittel enthält.

71. Essbares Paket nach Anspruch 3 oder 11, worin mindestens zwei verschiedene Arten von HPMC mit mindestens zwei verschiedenen scheinbaren Viskositäten verwendet werden.

72. Essbares Paket nach Anspruch 71, worin das CMC-Gehalt im Film zirka 5 Gew.-% beträgt.

73. Essbares Paket nach Anspruch 72, worin zwei verschiedene Arten von HPMC eine erste und zweite HPMC sind, und die erste HPMC eine scheinbare Viskosität von unter zirka 0,005 Pa·s (5 Centipoise) besitzt, und die zweite HPMC eine scheinbare Viskosität von zirka 0,1 Pa·s (100 Centipoise) oder zirka 0,045 Pa·s (45 Centipoise) besitzt, wo die scheinbare Viskosität eine 2%-ige wässrige HPMC-Lösung bei 20 °C beschreibt.

74. Essbares Paket nach Anspruch 73, worin die erste und zweite HPMC in einem Verhältnis von 70/30 bis 30/70 vorliegen.

75. Essbares Paket nach Anspruch 73, worin die erste und zweite HPMC in einem Verhältnis von 60/40 bis 40/60 vorliegen.

76. Essbares Paket nach Anspruch 74, worin die zweite HPMC eine scheinbare Viskosität von zirka 0,1 Pa·s (100 Centipoise) besitzt.

77. Essbares Paket nach Anspruch 75, worin die zweite HPMC eine scheinbare Viskosität von zirka 0,1 Pa·s (100 Centipoise) besitzt.

78. Essbares Paket nach Anspruch 73, worin die Mischung im Innern einen oder mehrere dieser Stoffe enthält: Xylitol, Erythrit, Mannit, Sorbit, Lactitol, Isomaltulose, Maltit und pulverförmiger hydrierter Glukosesirup.

79. Essbares Paket nach Anspruch 73, worin die Mischung im Innern weiterhin einen Geschmacksstoff und wahlweise ein Fließmittel enthält.

80. Essbares Paket nach Anspruch 73, worin der Film 0,0457 bis 0,0559 mm (1,8 mil bis 2,2 mil) dick ist.

81. Essbares Paket nach Anspruch 73, worin der Film 7 Gew.-% bis 9 Gew.-% Haftwasser enthält.

## Revendications

1. Sachet comestible comprenant un film auto-scellable externe d'environ 0,0381 à 0,0762 mm (1,5 à 3,0 millièmes de pouce (mil)) d'épaisseur, et une composition centrale, dans lequel la composition centrale comprend un ou plusieurs ingrédients qui ont une chaleur de dissolution négative et la composition centrale est séchée de façon à être stable au sein du film auto-scellable externe, et dans lequel le film est séché pour contenir 4 % à 12 % d'eau de rétention en poids et dans lequel le film comprend de l'hydroxypropylméthylcellulose (HPMC) et de la carboxyméthylcellulose (CMC) et un humectant, et dans lequel le film est susceptible de se sceller contre lui-même sous de la chaleur et de la pression et le sachet est stable à la température ambiante et susceptible de se dissoudre lorsqu'il est placé dans la bouche pour libérer la composition centrale.

2. Sachet comestible selon la revendication 1, dans lequel l'eau de rétention va de 6 % à 10 %.

3. Sachet comestible selon la revendication 2, dans lequel le film comprend en outre de la carraghénane.

4. Sachet comestible selon la revendication 2 à 3, dans lequel la composition centrale comprend du xylitol.

5. Sachet comestible selon la revendication 4, dans lequel la composition centrale comprend en outre un agent aromatisant et éventuellement un agent d'écoulement.

6. Sachet comestible selon la revendication 5, dans lequel la composition centrale comprend un agent aromatisant choisi parmi un ou plusieurs de : arômes de menthe verte, menthe poivrée, gaulthérie du Canada, cannelle, menthol, anis, thymol, eucalyptol, fruit, agrume, melon, ou baie.

7. Sachet comestible selon la revendication 6, dans lequel la composition centrale comprend un agent d'écoulement.

8. Sachet comestible selon la revendication 2 à 3, dans lequel le film comprend en outre de la glycérine et un ou plusieurs édulcorants.

9. Sachet comestible selon la revendication 2 à 7, dans lequel le film comprend en outre un agent aromatisant.

10. Sachet comestible selon la revendication 3, dans lequel le film comprend en outre un plastifiant comestible, et un agent aromatisant.

11. Sachet comestible selon la revendication 2, dans lequel le film comprend en outre de la carraghénane, de la cellulose microcristalline, et de la glycérine.

12. Sachet comestible selon la revendication 2, dans lequel la composition centrale comprend un ou plusieurs de : xylitol, érythritol, mannitol, sorbitol, lactitol, isomaltulose, maltitol ou sirop de glucose hydrogéné pulvérulent.

13. Sachet comestible selon la revendication 12, dans lequel la composition centrale comprend en outre un agent aromatisant et éventuellement un agent d'écoulement.

14. Sachet comestible selon la revendication 2 à 13, dans lequel la composition centrale comprend une combinaison d'ingrédients qui ne contient aucun sucre ou édulcorant en vrac.

15. Sachet comestible selon la revendication 2 à 13, dans lequel la composition centrale comprend un ou plusieurs sucres ou édulcorants en vrac.

16. Sachet comestible selon la revendication 2 à 13, dans lequel le film à une épaisseur de 0,0457 à 0,0559 mm (1,8 mil à 2,2 mil).

17. Sachet comestible selon la revendication 16, dans lequel le film contient 7 % à 9 % d'eau de rétention en poids.

18. Sachet comestible selon la revendication 17, qui est formé en une forme carrée ou rectangulaire.

19. Procédé de fabrication d'un sachet de confiserie susceptible de se dissoudre dans la bouche, comprenant la fourniture d'un film auto-scellable comestible, le formage du film en une forme de pré-sachet, le remplissage de la forme de pré-sachet avec une composition centrale, et le scellage de la forme de pré-sachet pour enfermer la composition centrale en appliquant de la chaleur et de la pression, dans lequel le film comprend de la HPMC et de la CMC et un humectant et est formé à une épaisseur de 0,0381 à 0,0732 mm (1,5 à 3,0 mil), et dans lequel le film contient 6 % à 10 % d'humidité de rétention, et dans lequel la composition centrale est séchée pour être stable au sein du film auto-scellable externe.

20. Procédé selon la revendication 19, dans lequel la composition centrale comprend du xylitol et éventuellement un agent d'écoulement.

21. Procédé selon la revendication 19, dans lequel la composition centrale comprend un ou plusieurs parmi : xylitol, érythritol, mannitol, sorbitol, lactitol, isomaltulose, et maltitol.

22. Procédé selon la revendication 19, dans lequel le film comprend une glycérine et éventuellement un agent aromatisant.

23. Procédé selon la revendication 19, dans lequel la composition centrale comprend un agent aromatisant.

24. Procédé selon la revendication 19, dans lequel le film comprend en outre de la carraghénane, de la cellulose microcristalline, et de la glycérine.

25. Procédé selon la revendication 24, dans lequel la composition centrale comprend un ou plusieurs de : xylitol, érythritol, mannitol, sorbitol, lactitol, isomaltulose, maltitol ou sirop de glucose hydrogéné pulvérulent.

26. Procédé selon la revendication 25, dans lequel la composition centrale comprend en outre un agent aromatisant et éventuellement un agent d'écoulement.

27. Procédé selon la revendication 19, dans lequel la composition centrale comprend une combinaison d'ingrédients qui ne contient aucun sucre ou édulcorant en vrac.

28. Procédé selon la revendication 19, dans lequel la composition centrale comprend un ou plusieurs sucres ou édulcorants en vrac.

29. Procédé selon la revendication 24, dans lequel le film à une épaisseur de 0,0457 à 0,0559 mm (1,8 mil à 2,2 mil).

30. Procédé selon la revendication 19, dans lequel le film contient 7 % à 9 % d'eau de rétention en poids.

31. Procédé selon la revendication 19, dans lequel le sachet est formé en une forme carrée ou rectangulaire.

32. Procédé de fabrication d'un sachet comestible, comprenant : la fourniture d'un film comestible, dans lequel le film a une teneur en humidité de rétention de 4 % à 12 %, dans lequel le film comprend de la HPMC, de la CMC et un humectant et est formé à une épaisseur de 0,0381 à 0,0762 mm (1,5 à 3,0 mil), et dans lequel le film est susceptible de s'auto-sceller contre lui-même ou un autre film ; le formage d'une forme de pré-sachet avec une ou plusieurs bandes de film ; le remplissage de la forme de pré-sachet avec une composition centrale, dans lequel la composition centrale est comestible ou conçue pour une utilisation ou consommation orale humaine ou vétérinaire et la composition centrale est séchée pour être stable au sein du film auto-scellable externe ; et le scellage de la forme de pré-sachet en appliquant de la chaleur et de la pression et/ou en appliquant un adhésif comestible à base d'eau au niveau des sites de scellage de façon à former un sachet fermé avec la composition centrale enclavée au sein du sachet fermé.

33. Procédé selon la revendication 31, dans lequel le film comprend en outre de la pectine.

34. Procédé selon la revendication 33, dans lequel le film comprend en outre un ou plusieurs parmi la glycérine, le propylène glycol, le monostéarate de glycérol, ou un plastifiant comestible.

35. Procédé selon la revendication 34, dans lequel le film comprend en outre un hydrocolloïde ou une gomme.

36. Procédé selon la revendication 32, dans lequel le film comprend en outre de la carraghénane, de la cellulose microcristalline, et de la glycérine.

37. Procédé selon la revendication 36, dans lequel le film comprend en outre un ou plusieurs ingrédients d'aromatisation et un ou plusieurs édulcorants.

38. Procédé selon la revendication 37, dans lequel le film comprend en outre un ou plusieurs ingrédients colorants.

39. Procédé selon la revendication 32, où la composition centrale comprend du xylitol.

40. Procédé selon la revendication 36, dans lequel la composition centrale comprend du xylitol et éventuellement un agent d'écoulement.

41. Procédé selon la revendication 32, dans lequel la composition centrale comprend un ou plusieurs parmi : xylitol, érythritol, mannitol, sorbitol, lactitol, isomaltulose, maltitol ou sirop de glucose hydrogéné pulvérulent.

42. Procédé selon la revendication 41, dans lequel la composition centrale comprend en outre un agent aromatisant et éventuellement un agent d'écoulement.

43. Procédé selon la revendication 32, dans lequel la composition centrale comprend une combinaison d'ingrédients sans aucun sucre ou édulcorant en vrac.

44. Procédé selon la revendication 32, dans lequel la composition centrale comprend un ou plusieurs sucres ou édulcorants en vrac.

45. Procédé selon la revendication 32, dans lequel la composition centrale comprend un agent aromatisant choisi parmi un ou plusieurs de : arômes de menthe verte, menthe poivrée, gaulthérie du Canada, cannelle, menthol, anis, thymol, eucalyptol, fruit, agrume, melon, et baie.

46. Procédé selon la revendication 32, dans lequel la composition centrale comprend un ou plusieurs parmi : menthol, thymol, eucalyptol, et salicylate de méthyle.

47. Procédé selon la revendication 32, dans lequel la composition centrale comprend un agent de rafraîchissement de l'haleine, un agent antibactérien, un agent pharmaceutique, ou un agent nutraceutique.

48. Procédé selon la revendication 32, dans lequel la composition centrale comprend un ou plusieurs parmi le chocolat noir, le chocolat au lait, le chocolat noir à faible teneur en sucre, le chocolat amer, le chocolat blanc ou la poudre de cacao.

49. Procédé selon la revendication 48, dans lequel le sachet est formé pour enfermer une composition centrale pour préparer une boisson lorsqu'il est placé dans de l'eau, du lait, de l'eau pétillante, ou un autre liquide.

50. Procédé selon la revendication 49, dans lequel la boisson est aromatisée au chocolat.

51. Procédé selon la revendication 32, dans lequel le sachet est formé en une forme conçue pour être placée dans la bouche.

52. Sachet comestible selon la revendication 1, dans lequel le film comprend de la HPMC, de la CMC, et un hydrocolloïde ou une gomme.

53. Sachet comestible selon la revendication 1, dans lequel la composition centrale comprend du xylitol.

54. Sachet comestible selon la revendication 53, dans lequel la composition centrale comprend un agent aromatisant choisi parmi un ou plusieurs de : arômes de menthe verte, menthe poivrée, gaulthérie du Canada, cannelle, menthol, anis, thymol, eucalyptol, fruit, agrume, melon, ou baie.

55. Sachet comestible selon la revendication 53, dans lequel le film comprend en outre un agent aromatisant et éventuellement un agent d'écoulement.

56. Sachet comestible selon la revendication 52, dans lequel le sachet comprend deux bandes de film comestible comprenant de la HPMC et de la CMC scellées ensemble par de la chaleur et de la pression à une période de temps souhaitée et/ou de l'eau et une quantité d'adhésif à base d'eau.

57. Sachet comestible selon la revendication 56, dans lequel le film comestible comprend en outre de la carraghénane, de la glycérine, et un ou plusieurs édulcorants.

58. Sachet comestible selon la revendication 57, dans lequel le film comestible comprend en outre un agent aromatisant.

59. Sachet comestible selon la revendication 58, dans lequel la composition centrale comprend un ou plusieurs parmi : xylitol, érythritol, mannitol, sorbitol, lactitol, isomaltulose, et maltitol.

60. Sachet comestible selon la revendication 58, dans lequel la composition centrale comprend un ou plusieurs parmi : menthol, thymol, eucalyptol, et salicylate de méthyle.

61. Sachet comestible selon la revendication 58, dans lequel la composition centrale comprend un ou plusieurs parmi le chocolat noir, le chocolat au lait, le chocolat noir à faible teneur en sucre, le chocolat amer, le chocolat blanc ou la poudre de cacao.

62. Sachet comestible selon la revendication 58, dans lequel la composition centrale comprend un ou plusieurs parmi : érythritol, mannitol, sorbitol, lactitol, isomaltulose, maltitol, sirop de glucose hydrogéné pulvérulent, menthe verte, menthe poivrée, gaulthérie du Canada, cannelle, menthol, anis, thymol, eucalyptol, arômes de fruit ou de baie, dextrose, saccharose, fructose, maltose, dextrine, sucre inversé, lévulose, galactose, solides de sirop de maïs, un agent de rafraîchissement de l'haleine, un agent antibactérien, un agent pharmaceutique, un agent vétérinaire, et un agent nutraceutique.

63. Sachet comestible selon la revendication 1, dans lequel le film comestible comprend un ou plusieurs parmi la CMC, la HPMC, la gomme guar, la gomme de caroube, la gomme de xanthane, des gommes hydrolysées, la carraghénane, le tamarin, l'agar-agar, le konjac, l'arabinogalactane, l'arabinogalactane de mélèze, le bêtaglucane, l'algine, le propylène glycol, la polyvinylpyrrolidone, un copolymère méthacrylate, un copolymère carboxyvinyle, le lévane, l'elsinane, la pullulane, la pectine, le curdlan, le chitosan, la gomme arabique, l'amidon de maïs, l'amidon de maïs cireux, l'amidon de pomme de terre à haute teneur en amylose, l'amidon de pomme de terre, l'amidon de tapioca, l'amidon de riz, l'amidon de blé, des amidons modifiés, des amidons modifiés à l'acide, des amidons blanchis, des amidons oxydés, des amidons estérifiés, des amidons éthérifiés, des amidons réticulés, et une protéine ou des compositions de protéines telles que l'albumen, la gélatine, la caséine, des sels de caséine, le lactosérum, le gluten de blé, la zéine, et des isolats de soja.

64. Sachet comestible selon la revendication 63, dans lequel le film contient un ou plusieurs agents aromatisants.

65. Sachet comestible selon la revendication 1, dans lequel le film est essentiellement dépourvu d'un quelconque agent aromatisant.

66. Sachet comestible selon la revendication 1, dans lequel le film a un taux d'eau de rétention d'environ 5 % à environ 10 %.

67. Sachet comestible selon la revendication 1, dans lequel la composition centrale comprend un ou plusieurs arômes épicés.

68. Sachet comestible selon la revendication 1, dans lequel la composition centrale comprend un ou plusieurs ingrédients ou compositions à base de matière grasse.

69. Sachet comestible selon la revendication 1, dans lequel la composition centrale comprend un ou plusieurs agents d'hydratation de la bouche.

70. Sachet comestible selon la revendication 1, dans lequel la composition centrale comprend un ou plusieurs agents de rafraîchissement de l'haleine.

71. Sachet comestible selon la revendication 3 ou 11, dans lequel au moins deux types différents de HPMC sont utilisés avec au moins deux viscosités apparentes différentes.

72. Sachet comestible selon la revendication 71, dans lequel le taux de CMC dans le film est d'environ 5 % en poids.

73. Sachet comestible selon la revendication 72, dans lequel deux types différents de HPMC sont une première et une deuxième HPMC, et la première HPMC a une viscosité apparente d'environ moins de 0,005 Pa·s (5 centipoises), et la deuxième HPMC a une viscosité apparente d'environ 0,1 Pa·s (100 centipoises) ou environ 0,045 Pa·s (45 centipoises), où la viscosité apparente représente une solution aqueuse à 2% de HPMC à 20°C.

74. Sachet comestible selon la revendication 73, dans lequel les première et deuxième HPMC sont présentes dans le rapport de 70/30 à 30/70.

75. Sachet comestible selon la revendication 73, dans lequel les première et deuxième HPMC sont présentes dans le rapport de 60/40 à 40/60.

76. Sachet comestible selon la revendication 74, dans lequel la deuxième HPMC a une viscosité apparente d'environ 0,1 Pa·s (100 centipoises).

77. Sachet comestible selon la revendication 75, dans lequel la deuxième HPMC a une viscosité apparente d'environ 0,1 Pa·s (100 centipoises).

78. Sachet comestible selon la revendication 73, dans lequel la composition centrale comprend un ou plusieurs parmi : xylitol, érythritol, mannitol, sorbitol, lactitol, isomaltulose, maltitol ou sirop de glucose hydrogéné pulvérulent.

79. Sachet comestible selon la revendication 73, dans lequel la composition centrale comprend en outre un agent aromatisant et éventuellement un agent d'écoulement.

80. Sachet comestible selon la revendication 73, dans lequel le film à une épaisseur de 0,0457 à 0,0559 mm (1,8 mil à 2,2 mil).

81. Sachet comestible selon la revendication 73, dans lequel le film contient 7 % à 9 % d'eau de rétention en poids.
